(19) Europäisches Patentamt
European Patent Office
Office européen des brevets

(11) Publication number: **0 226 896 B1**

(12) **EUROPEAN PATENT SPECIFICATION**

(45) Date of publication of patent specification: **15.09.93**

(51) Int. Cl.$^5$: **C07D 493/08**, //A61K31/35, (C07D493/08,313:00,311:00)

(21) Application number: **86116752.6**

(22) Date of filing: **02.12.86**

The file contains technical information submitted after the application was filed and not included in this specification

(54) **Lankacidin derivatives and production thereof.**

(30) Priority: **05.12.85 JP 274256/85**
**06.10.86 JP 238788/86**

(43) Date of publication of application:
**01.07.87 Bulletin 87/27**

(45) Publication of the grant of the patent:
**15.09.93 Bulletin 93/37**

(84) Designated Contracting States:
**AT BE CH DE ES FR GB GR IT LI LU NL SE**

(56) References cited:
**EP-A- 0 091 781**
**EP-A- 0 091 782**
**DE-A- 2 014 277**
**GB-A- 2 088 715**

**THE JOURNAL OF ANTIBIOTICS, 28(5), May 1975, pages 390-394, Japan antibiotics research association, Tokyo, JP; K. NAKAHAMA et al.: "Studies on lankacidin-group (T-2636) antibiotics. X microbial conversion of lankacidin-group antibiotics"**

**THE JOURNAL OF ANTIBIOTICS, 26(11), November 1973, pages 647-657, Japan antibiotics research association, Tokyo, JP; S. HARADA et al.: "Studies on lankacidin-group (T-2636) antibiotics. VII. structure-activity relationships of lankacidin-group antibiotics"**

**ACTA CRYSTALLOGRAPHICA, volume B27, part 1, 15th January 1971, pages 236-241; M. URAMOTO et al.: "The mophenylsulphonylhydrazone" * whole document ***

(73) Proprietor: **Takeda Chemical Industries, Ltd.**
**1-1, Doshomachi 4-chome**
**Chuo-ku, OSAKA(JP)**

(72) Inventor: **Minamida, Isao**
**5-91 Fushimidai 2-chome**
**Inagawa-cho Kawabe-gun Hyogo 666-02(JP)**
Inventor: **Hashimoto, Naoto**
**4-15, Tsukumodai 4-chome**
**Suita Osaka 565(JP)**

(74) Representative: **von Kreisler, Alek, Dipl.-Chem. et al**
**Patentanwälte, Von Kreisler-Selting-Werner,**
**Postfach 10 22 41, Bahnhofsvorplatz 1**
**D-50462 Köln (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

## Description

The present invention relates to novel lankacidin derivatives having antimicrobial activities and a method of preparing them.

Lankacidins are produced by cultivation of strains belonging to the genus Streptomyces and have structural formula (i) or (ii).

(i)

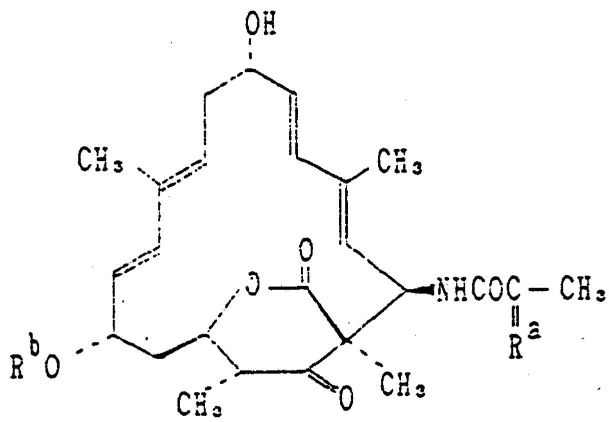

(ii)

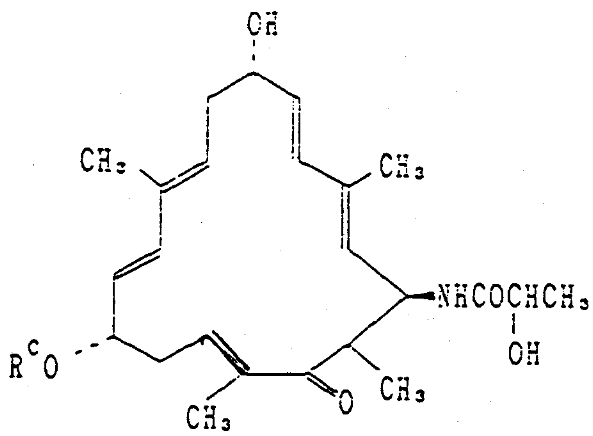

Lankacidin A is a compound of the general formula (i) wherein $R^a$ : O, $R^b$ : $COCH_3$, lankacidin C is a compound of the general formula (i) wherein $R^a$ : O, $R^b$ : H, lankacidinol A is a compound of the general formula (i) wherein

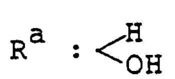 ,

$R^b$ : $COCH_3$ and lankacidinol is a compound of the general formula (i) wherein

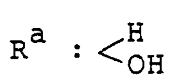 ,

$R^b$ : H and lankacyclinol is a compound of the general formula (ii) wherein $R^c$ : H and lankacyclinol A is a compound of the general formula (ii) wherein $R^c$ : $COCH_3$. Among derivatives of the above-mentioned lankacidins, for example, esters at 8- or/and 14 positions of lankacidin C [Acta Cryst., B27, P.236 (1971) ; J.

Antibiotics, 26, P.647 (1973)] 3-amido modified derivatives [Antimicrobial Agents and Chemotherapy, 25 pp.226~233 (1984)] and acyloxy derivatives at 8- or/and 14-positions [The Journal of Antibiotics, 26, p.647 (1973)] have been known.

While, as stated above, lankacidins have been produced, the present invention is directed to production of novel derivatives of these lankacidins.

The present inventors synthesized from the above-mentioned lankacidins a variety of derivatives, and studied pharmacological activities thereof, and found that these derivatives have excellent antimicrobial activities.

Based on these findings, the present inventors conducted further extensive studies and have completed the present invention.

The present invention relates to compounds of the general formula

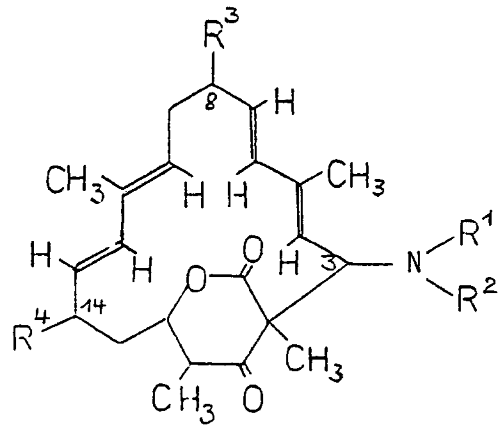

wherein

(i)

$R^1$ stands for a hydrogen atom,

$R^2$ stands for a group of the formula:

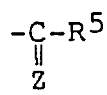

wherein Z stands for oxygen or sulfur, and $R^5$ stands for an $C_1$-$C_5$-alkanoyl group, an $\alpha$-hydroxy$C_{1-4}$alkyl group, or

(ii)

$R^1$ and $R^2$, taken together, stand for a group of the formula

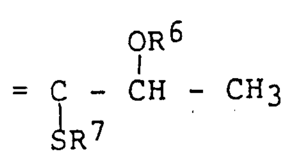

wherein $R^6$ stands for a $C_{1-5}$ alkanoyl group and $R^7$ stands for a $C_{1-4}$alkyl group;

$R^3$ is

(1) -OCOO-$C_{1-4}$alkyl,

(2) -OCOS-$C_{1-4}$alkyl substituted with $\omega$-$C_{2-4}$alkanoylamino,

(3) -OCOR$^{14}$ wherein $R^{14}$ is a $C_{1-4}$alkyl group substituted at the terminus with (a) di($C_{1-4}$alkyl)amino or (b) a 5 to 8 membered heterocyclic group containing 1 to 4 hetero atoms of oxygen, sulfur or nitrogen, and having optionally an intermediate sulfur atom;

(4) $-OCONR^{15}R^{16}$ wherein $R^{15}$ and $R^{16}$ respectively are a $C_{1-5}$alkyl group or together form a heterocyclic group containing nitrogen atoms which may be substituted by (1) $C_{1-4}$alkyl or (2) pyridyl;
$R^4$ is hydroxy or a $C_{1-4}$alkanoyloxy group;
or a salt thereof.

The invention relates further to compounds selected from O(8)-(4-benzylpiperazino)carbonyl-lankacidin C,
3-(2-acetoxyacrylamido)lankone 8, 14-diacetate,
O(8)-(2-acetamidoethylthio)methoxycarbonyl lankacidin C,
lankacidin C 8-(2-chloroethyl)aminoacetate,
lankacidin C 8-(2-fluoroethyl)aminoacetate,
O(8)-[2-(2-pyridyl)ethylamino]carbonyl lankacidin C,
O(8)-[(2-pyridyl)methylamino]carbonyl lankacidin C,
lankacidin C 8-propylaminoacetate,
lankacidin C 8-isopropylaminoacetate,
lankacidin C 8-(4-(4-pyridyl)piperazino)acetate,
O(8)-[(5-methoxymethyl-1,3,4-thiadiazol-2-yl)thio] methoxycarbonyl-lankacidin C,
O(8)-(4-pyridylmethylamino)carbonyl-lankacidin C,
lankacidin C 8-azidoacetate,
8-dehydroxy-8-(dimethyl aminoethyl-1H-tetrazol-5-yl)thio-lankacidinC
O(8)-[(1-methyl-1H-tetrazol-5-yl)thio]methoxy carbonyl-lankacidin C,
lankacidin C 8-[(1-methyl-1H-tetrazol-5-yl)thio]-acetate,
lankacidin C 8-[4-(2-pyridyl)piperazino]acetate,
O(8)-ethylaminocarbonyl lankacidin C,
O(8)-morpholinocarbonyl lankacidin C,
O(8)-(3-pyridyl)methylaminocarbonyl-lankacidin C,
or a salt thereof.

The invention is also related to a method for producing the compound which comprises subjecting a compound of the formula:

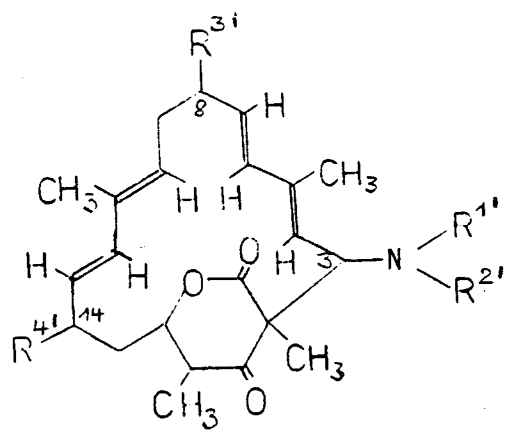

wherein $R^{1'}$, $R^{2'}$, $R^{3'}$ and $R^{4'}$ are respectively the same as $R^1$, $R^2$, $R^3$ and $R^4$ defined above or the groups convertible thereto, to alkylation, acylation, hydrolysis, thioamidation, halogenation, azidation, reaction with amines, reaction with a thiol or a salt, and/or reaction with isocyanate.

The $C_{1-4}$ alkyl groups and $C_{1-5}$ alkyl groups respectively may be straight-chain or branched ones, which are exemplified by methyl, ethyl, propyl, isopropyl, butyl, isobutyl, sec-butyl, tert.-butyl and pentyl.

The 5-8 - membered heterocyclic groups containing 1-4 hetero atoms of oxygen, sulfur or nitrogen, are exemplified by thienyl, furyl, pyrrolyl, pyridyl, oxazolyl, thiazolyl, pyrazolyl, imidazolyl, isooxazolyl, isothiazolyl, 1,2,4-oxadiazolyl, 1,3,4-oxadiazolyl, 1,2,4-thiadiazolyl, 1,3,4-thiadiazolyl, 1,2,3-thiadiazolyl, 1,2,3-triazolyl, 1,2,4-triazolyl, 1,3,4-triazolyl, tetrazolyl, N-oxido-pyridyl, pyrimidinyl, N-oxido-pyrimidinyl, pyridazinyl, pyrazinyl, N-oxido-pyridazinyl, benzofuryl, benzothiazolyl, benzoxazolyl, triazinyl, tetrazolo[1,5-b] pyridazinyl, triazolo[4,5-b]pyridazinyl, oxoimidazolinyl dioxotriazinyl, pyrrolidinyl, piperidinyl, pyranyl, thiopyranyl, 1,4-oxadinyl, morpholinyl, 1,4-thiadinyl, 1,3-thiazinyl, piperazinyl.

$C_{1-4}$ alkylthio groups are exemplified by methyltio, ethylthio, propylthio and butylthio.

Di-$C_{1-4}$alkylamino groups are exemplified by dimethylamino, diethylamino, dipropylamino, dibutylamino, N-methyl-N-ethylamino, N-methyl-N-propylamino and N-methyl-N-butylamino.

$C_{1-4}$alkoxycarbonyl groups are exemplified by methoxycarbonyl, ethoxycarbonyl, propoxycarbonyl, isopropoxycarbonyl, butoxycarbonyl, tert-butoxycarbonyl and isobutoxycarbonyl.

$C_{1-5}$ alkanoyl groups are exemplified by formyl, acetyl, propionyl, butyryl, and pivaloyl.

$C_{1-4}$ alkanoyloxy groups are exemplified by formyloxy, acetoxy, butyryloxy, and pivaloyloxy.

$C_{1-4}$alkoxycarbonyloxy groups are exemplified by methoxycarbonyloxy, ethoxycarbonyloxy and tert-butoxycarbonyloxy. Description of each of the afore-mentioned groups will be given hereafter more concretely.

The group represented by the formula

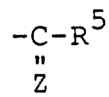

is exemplified by 1,2-dioxopropyl, 2-oxo-1-thioxopropyl, 1,2-dioxobutyl, 2-oxo-1-1-thioxobutyl, 1,2-dioxopentyl, 2-oxo-1-thioxopentyl, 2-hydroxy-1-thioxopropyl, 2-hydroxypropionyl, 2-hydroxy-1-thioxopropyl,
When $R^1$ and $R^2$ , taken together, stand for the formula;

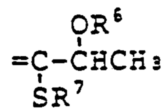

(wherein $R^6$ and $R^7$ are of the same meaning as defined in the foregoing), practical examples of $R^6$ are, among others, acetyl, propyl, butyryl, pivaloyl and pentanoyl. As $R^7$ are practically mentioned, among others, methyl, ethyl, propyl and butyl.

Practical examples of $R^3$ are, methoxycarbonyloxy, ethoxycarbonyloxy, propoxycarbonyloxy, butoxycarbonyloxy, tert-butoxycarbonyloxy, formyloxy, acetoxy, propionyloxy, butyryloxy, isobutyryloxy, pentanoyloxy, pivaloyloxy, (2-dimethylaminoethylthio)carbonyloxy, (2-acetylaminoethylthio)-carbonyloxy, (3-dimethylamino)propanoyloxy, (3-methylthio)propanoyloxy, (2-dimethylaminoethyl)thioacetoxy, (2-dimethylaminoethyl)thio)acetoxy, [(diethylaminoethyl)thio]acetoxy, dimethylaminoacetoxy, diethylaminoacetoxy, di-iso-propylaminoacetoxy, morpholinoacetoxy, 3-dimethylaminopropanoyloxy, 4-dimethylaminobutyryloxy, N,N-dimethylcarbamoyloxy, N,N-diethylcarbamoyloxy, N,N-dipropylcarbamoyloxy, N,N-dinutylcarbamoyloxy, N-ethyl-N-methylcarbamoyloxy, pyrrolidinocarbonyloxy, piperidinocarbonyloxy, morpholinocarbonyloxy and N'-methylpiperazinocarbonyloxy.

Practical examples for $R^4$ are hydroxy, acetoxy, propionyloxy, butyryloxy, and isobutyryloxy.

Compounds of the present invention can be produced, for example, by subjecting a compound representable by the general formula

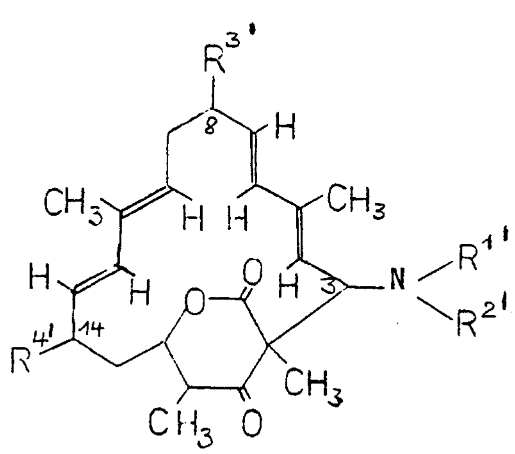

wherein $R^{1'}$, $R^{2'}$, $R^{3'}$ and $R^{4'}$ are respectively the same as $R^1$, $R^2$, $R^3$ and $R^4$ defined above or the groups convertible thereto, to alkylation, acylation, hydrolysis, thioamidation, halogenation, azidation, reaction with amines, reaction with a thiol or a salt, and/or reaction with isocyanate.

More concretely, a compound representable by the general formula;

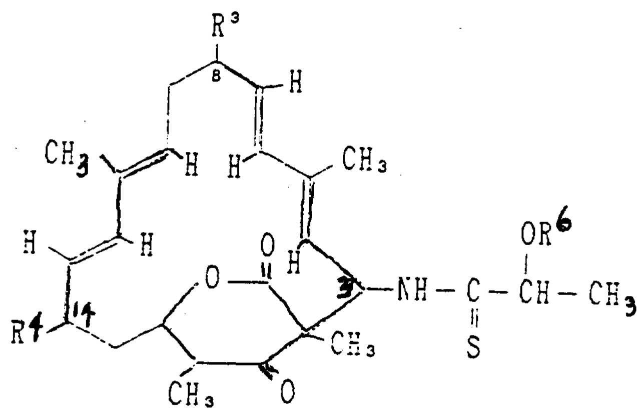

[1-2]

[wherein $R^3$, $R^4$ and $R^6$ are of the same meanings as defined above] is subjected to alkylation to produce a compound representable by the general formula;

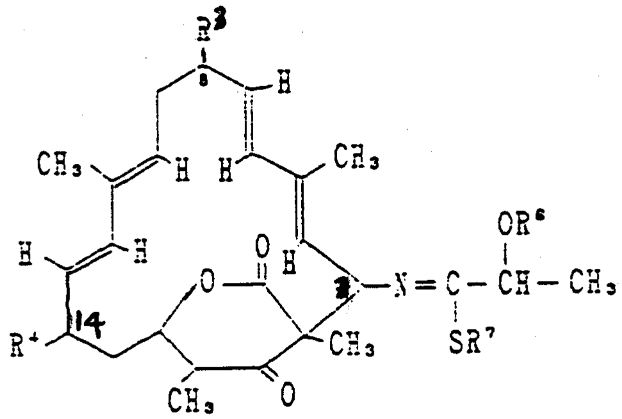

[2]

[$R^3$, $R^4$, $R^6$ and $R^7$ are of the same meaning as defined above], the compound [2]is subjected to (i) acylation, followed by hydrolysis, or (ii) hydrolysis, followed by acylation, to produce a compound representable by the general formula;

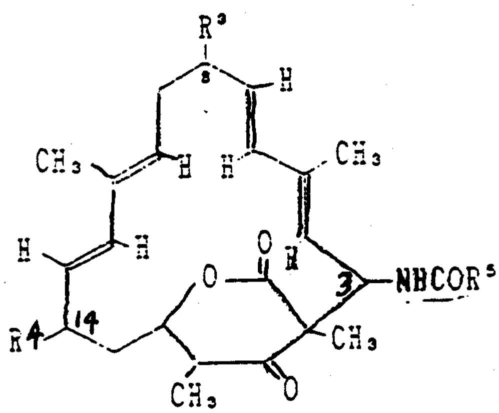

[1-3]

[wherein $R^3$, $R^4$ and $R^5$ and of the same meanings as defined above], a compound representable by the general formula;

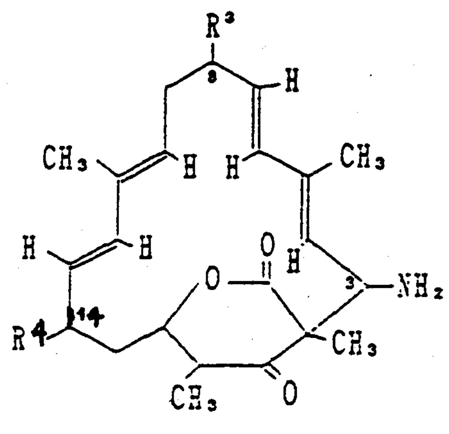

[3]

[wherein $R^3$ and $R^4$ are of the same meaning as defined above, is subjected to acylation, then, upon necessity, to thioamidation and to alkylation to produce a compound representable by the general formula;

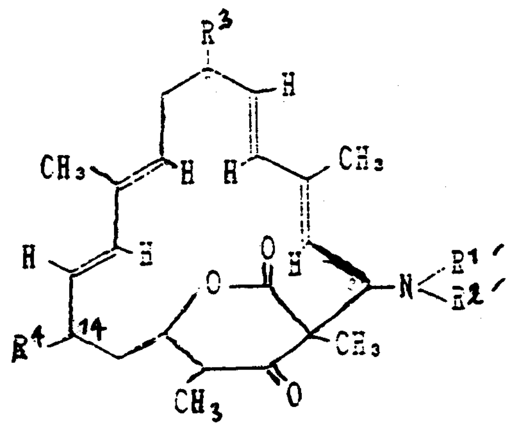

[1-4]

[wherein $R^{1'}$, $R^{2'}$, $R^3$ and $R^4$ are of the same meanings as defined above], or a compound representable by the general formula;

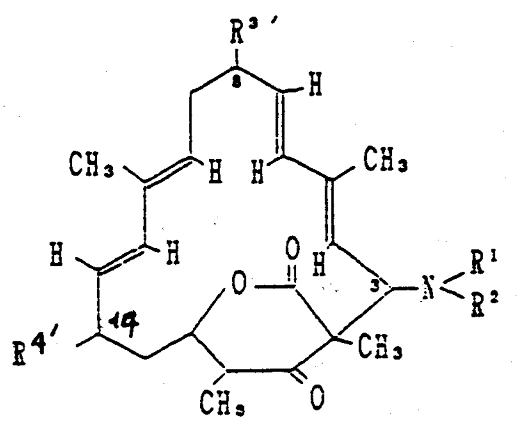

[1-6]

[wherein $R^1$, $R^2$, $R^3$ and $R^{4'}$ are of the same meanings as above) is subjected to halogenation, azidation or reaction with amines, reaction with thiol or a salt and/or reaction with isocyanate, to produce the compound (1).

The compound [2], which is a compound of the general formula [1] wherein $R^1$ and $R^2$ combinedly stand for a group representable by the formula ;

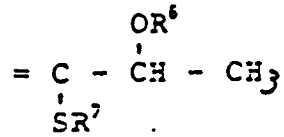

(wherein $R^6$ and $R^7$ are of the same meaning as defined above), can be prepared by, for example, the reaction process as shown below :

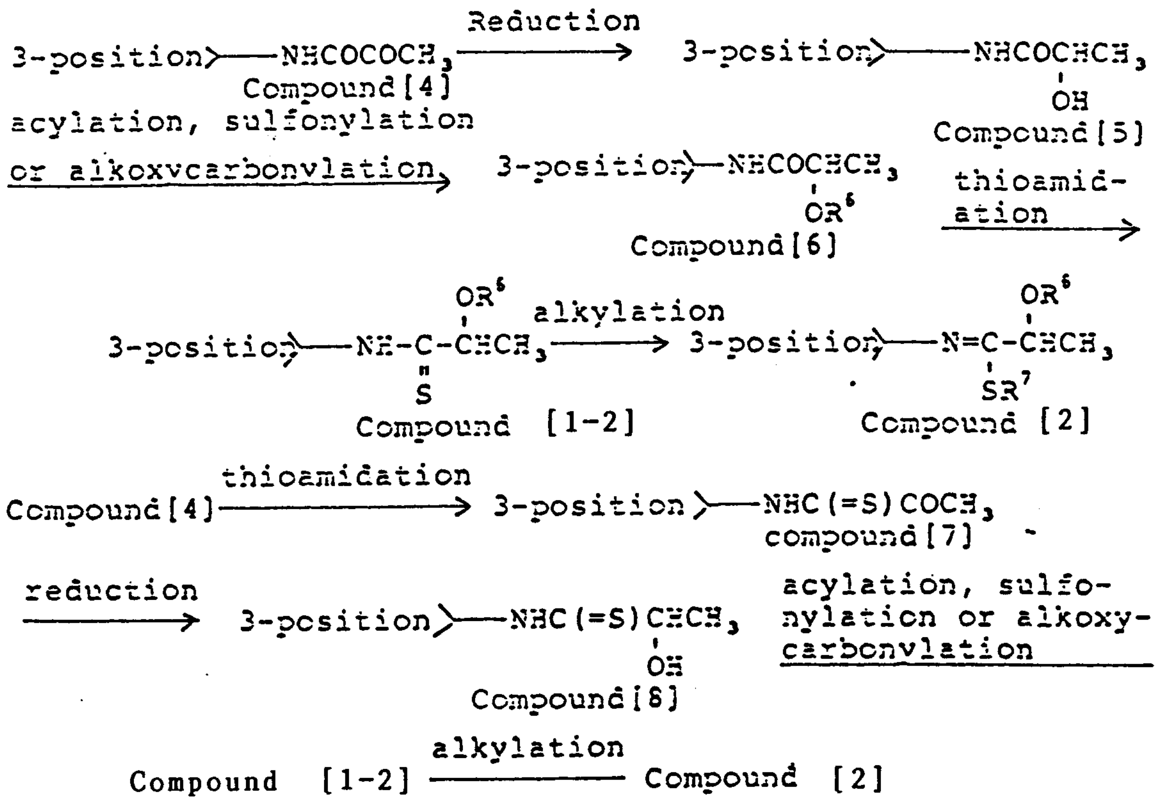


Namely, a compound [1] wherein the group

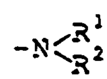

is pyruvoylamino, which is the compound [4], is subjected to reduction to give 2'-hydroxy compound [5], which is subjected to acylation, sulfonylation or alkoxycarbonylation to give the compound [6], which is subjected to thioamidation to give the compound [1-2], then the compound [1-2] is subjected to alkylation or the compound [4] is subjected to thioamidation to give the compound [7], which is subjected to reduction to give the compound [8], which is subjected to acylation, sulfonylation or alkoxylation to give the compound [1-2], followed by subjecting the same to alkylation to obtain the compound [2].

The reduction of the compound [4]—the compound [5] or the reduction of the compound [7]—the compound [8] is preferably conducted in general bringing the starting material into contact with a borohydride compound. The borohydride compound is exemplified by sodium borohydride, lithium borohydride or sodium cyanoborohydride. In general, the reaction is preferably conducted in a solvent such as alcohols e.g. methanol or ethanol, ethers e.g. tetrahydrofuran, 1,4-dioxane, 1,2-dimethoxyethane or 2-methoxyethylether or a mixture of these solvents with water. It is not advantageous to use a large excess volume of the reaction reagent, because there are other positions at which the reaction occurs with the reagent (e.g. 18-position), and it is preferable in general to employ about 1~2 times as much of the theoretical volume, even when taking the volume to be consumed by the reaction with the solvent then

used. The reaction temperature is suitably selected within the range of from about -70 °C to room temperatures, preferably within the range of from about -30 °C to about 0 °C. The reaction time ranges from about one minute to three hours.

The reaction of the compound [5] → the compound [6] or acylation, sulfonylation or alkoxycarbonylation of the compound [8] → the compound [1-2] can be conducted by a per se conventional method or a method analogous thereto.

The acylating agent employed for the acylation is exemplified by acyl halide containing an acyl group shown by $R^6$, acid anhydride. The sulfonylating agent for the sulfonylation is exemplified by, among others, sulfonyl halide containing a sulfonyl group shown by $R^6$ or sulfonic anhydride. The alkoxycarbonylating agent for the alkoxycarbonylation is exemplified by alkoxycarbonyl halide containing an alkoxycarbonyl group shown by $R^6$ or bicarbonate ester. As the halogen in the above-mentioned halide, bromine and chlorine are especially preferably. The amount of the reagent is equimolar or more, preferably about 1~5 molar equivalents. In the above acylation, when an acid anhydride is employed as the acylating agent may be used in an excess amount. As the solvent for the reaction, any one which is capable of dissoving the compound [5] or the compound [8] and the reaction reagent can be employed, and preferably exemplified by dichloromethane, chloroform, dichloroethane, tetrahydrofuran, dioxane, N,N-dimethylformamide, N,N-dimethylacetamide, dimethylsulfoxide, hexamethylphosphorotriamide, pyridine. The reaction temperature ranges from about -50 °C to 30 °C, and the reaction time ranges from about 0.1 to 24 hours. By allowing amines such as triethylamine, dimethylaminopyridine, pyridine, N,N-dimethylaniline, N,N-diethylaniline, to coexist in the reaction system, the reaction time can be shortened and the yield can be improved while controlling possible side reactions.

The reaction of the compound [6]→ the compound [1-2] or the thioamidation of the compound [4]→the compound [7] can be conducted by employing for example phosphorus pentasulfide or Lawesson's reagents. The Lawesson's reagents are exemplified by 2,4-bis(4-methoxyphenyl)-1,3-dithia-2,4-dip hosphethane-2,4-disulfide, 2,4-bis(4-phenoxyphenyl)-1,3-dithia-2,4-diphosphethane-2,4-disulfide, 2,4-bis(4-methylthiophenyl)-1,3-dithia-2,4-diphosphethane-2,4-disulfide, 2,4-bis(4-phenylthiophenyl)-1,3-dithia-2,4-diphosphethane-2,4-disulfide. The amount of the reagents for the reaction is about 0.5~30 molar equivalent in general, but, when the reaction is conducted at a high temperature, the amount is desirably within the range of about 0.5~3 molar equivalent. The solvent to be employed for the reaction is exemplified by dichloromethane, tetrahydrofuran, 1,4-dioxane, hexamethylphosphorotriamide, pyridine. The reaction temperature ranges from about 20 °C to 110 °C, and the reaction time is about 0.1 24 hours.

The alkylation from the compound [1-2] to the compound [2] is conducted by bringing the compound [1-2] into contact with an alkylating agent. The reagent to be employed is exemplified by alkyl halides such as alkyl iodide, alkyl bromide and alkyl chloride, dialkylsulfuric acid, Meerwein reagent. The amount of a reagent to be used for the reaction, when the reagent is alkyl halide or dialkyl sulfuric acid, is 1 molar equivalent to a large excess, desirably about 3 molar equivalent to a large excess. When a Meerwein reagent is employed, use of about 1-2 molar equivalent brings about a preferably result. The solvent to be employed for the reaction is exemplified by dichloromethane, chloroform, tetrahydrofuran, ethyl acetate, and, when an alkyl halide or dialkyl sulfuric acid is employed as the alkylating agent, it is desirable to have an inorganic base such as sodium hydrogencarbonate, sodium carbonate, potassium hydrogencarbonate or potassium carbonate coexist, and it is also desirable to have water to coexist together with the above-mentioned solvent so as to dissolve these inorganic bases. The reaction temperature is within the range of about 0.5 hour ~ 10 days. When a Meerwein reagent is used as the alkylating agent, the reaction proceeds quickly and completes generally in about 5 hours. Incidentally, when a Meerwein reagent is used as the alkylating agent, a salt of the compound [2] is obtained, and, in case of isolating the compound [2], it is necessary to neutralize this salt with a base (e.g. sodium hydrogencarbonate, potassium hydrogencarbonate, sodium carbonate, potassium carbonate, sodium dihydrogenphosphate, potassium dihydrogenphosphate, sodium acetate).

Acylation of the compound [2] can be conducted by bringing the compound [2] into contact with an acylating agent containing a group shown by $R^5$. The acylating agent is exemplified by acid halide, a mixed acid anhydride, activated ester, and acid chloride and acid bromide are especially preferablly employed. The amount of the acid halide is about 1-3 molar equivalent. The reaction is conducted preferably in a solvent, and the solvent is exemplified by acetic acid esters such as methyl acetate, ethyl acetate, propyl acetate, butyl acetate, halogenated hydrocarbons such as dichloromethane, chloroform, ethers such as ethylether, 1,4-dioxane, tetrahydrofuran, N,N-dimethylformamide, N,N-dimethylacetamide, dimethyl sulfoxide, hexamethyl phosphorotriamide. The reaction temperature is about 0 °C ~ about 80 °C, and it is convenient to conduct the reaction around room temperature. The reaction time is about 1 hour ~ 24 hours. The subsequent hydrolysis proceeds by the addition of water after completion of the above-mentioned

acylation, and, depending on cases, the hydrolysis completes by the presence of moisture contained in the solvent used when the acylation is conducted. In case of adding water, the volume is, for example, 1 ~ 10 molar equivalent. The reaction temperature is about 0 °C ~ 50 °C and the reaction time is about 1 hour ~ 24 hours to bring about preferable results.

By-products formed when the compound [1-3] is prepared from the above-mentioned compound [2] can be eliminated by conventional means such as chromatography, recrystallization, reprecipitation.

For preparing the compound [1-4] by acylating the compound [3], and, upon necessity, by subjecting the acylated compound to thioamidation, firstly the compound [2] is subjected to acid hydrolysis, then the resultant compound [3] is subjected to acylation.

Acid hydrolysis of the compound [2] is conducted by bringing the compound [2] into contact with an inorganic acid e.g. hydrochloric acid, sulfuric acid, hydrobromic acid, hydriodic acid, hydrofluoric acid, nitric acid, perchloric acid, chromic acid, periodic acid or hydrofluoric acid, or an organic acid such as methanesulfonic acid, benzenesulfonic acid or toluenesulfonic acid in the presence of water. The reaction is conducted preferably in an organic solvent so as to dissolve the compound [2]. The solvent to be used is exemplified by acetone, tetrahydrofuran, 1,4-dioxane, duchloromethane, chloroform, dichloroethane, methanol, ethanol. The reaction temperature ranges from about 0 °C to about 50 °C, and it is convenient to conduct the reaction at room temperatures. The reaction time varies with reaction temperatures,acid concentrations and kinds of organic solvent then employed, and it ranges from 0.5 hour to 24 hours. The compound [3] produced by acid hydrolysis of the compound [2], the former being unstable, is desirably subjected to acylation without isolation to lead to the compound [1-3]. More concretely, the desirable method comprises allowing an acylating agent to coexist when the compound [2] is subjected to acid hydrolysis then acylating the compound [3] immediately to lead to the compound [1-3].

The acylation of the compound [3] can be conducted in a manner similar to the afore-mentioned reaction for acylating the compound [2].

The reaction in case of subjecting the compound obtained by the said acylation to thioamidation upon necessity can be conducted in a manner similar to the reaction in case of subjecting the afore-mentioned compound [6] or the compound [4] to thioamidation.

Compound [45], which is a compound of the general formula [1] wherein one of $R^1$ and $R^2$ is hydrogen and the other is

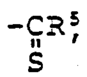

can be prepared by subjecting corresponding 3-amido compound [1-3] to thioamidation. This conversion reaction of Compound [1-3] → Compound [45] can be conducted in a manner similar to the above-mentioned conversion reaction of Compound [6] → Compound [1-2].

Further, the above-mentioned conversion reaction conducted on corresponding 3-amido compound can also be applied to a 3-thiamido compound, and by these conversion reactions any other derivatives can be prepared.

Compound [46], which is a compound of the general formula [1] wherein $R^4$ is hydroxyl, can be prepared, for example, by liberating its carbonic acid ester, carboxylic acid ester, phosphoric acid ester, sulfonic acid ester or ether compound as described later.

In case of using the carbonic acid ester or carboxylic acid ester for the purpose of liberating later, in other words, in case of introducing it as the protective group of the hydroxyl group of $R^4$, the liberation can be performed more easily. Such carbonic acid ester and carboxylic acid ester are exemplified by, besides benzyloxycarbonyloxy, 2,2,2-trichloroethoxycarbonyloxy, aryloxycarbonyloxy, chloroacetyloxy, those described on literature references [for example, "Protective Groups in Organic Synthesis" written by Theodora W. Greene, published by John Wiley & Sons, New York, 1981].

Ether is usually difficult to be removed. Therefore, when ether is used for the purpose of obtaining hydroxyl compound, the ether is limited to that usable as a protective group. As such ether compounds, use is made of, besides trimethylsilylether, tert-butyldimethylsilylether, tert-butyldiphenylether, methylether, methoxyethoxyether, those described on the literature reference cited above.

Removal of the carbonic acid ester, carboxylic acid ester, phosphoric acid ester or sulfonic acid ester can be conducted by a per se known method or a method analogous thereto. More specifically, the reaction is hydrolysis and can be conducted under conditions similar to conventional ester hydrolysis.

Namely, the hydrolysis can be conducted generally in a solvent (singly or in admixture of one or more solvents), exemplified by water, alcohols (e.g. methanol, ethanol, propanol, butanol, diethylene glycol, 2-methoxyethanol), ketones (e.g. acetone), ethers (e.g. tetrahydrofuran, dioxane, dimethoxyethane), amides (e.g. dimethylformamide, dimethylacetamide, hexamethylphosphorotriamide, sulfoxides (e.g. dimethylsulfoxide), sulfones (e.g. sulfolane), carboxylic acids (e.g. formic acid, acetic acid), by using an acid (e.g. mineral acids such as hydrochloric acid, hydrobromic acid, sulfuric acid, organic acids such as p-toluenesulfonic acid, strongly acid ion-exchange resin) or a base (e.g. sodium hydroxide, potassium hydroxide, potassium carbonate, sodium hydrogencarbonate, barium hydroxide, calcium carbonate, sodium methoxide, ammonia), and the hydrolysis by using a base is preferable. The amount of a base is about 1 ~ 10 times as much moles, preferably about 1.2 ~ 4 times as much moles. The reaction temperature and time greatly depend on the kinds of acyl moiety in the ester group then employed, they are respectively about -20 °C ~ 70 °C, preferably about -5 °C ~ 30 °C and about 0.1 ~ 24 hours, preferably about 0.1 ~ 3 hours.

When an ether compound is employed as the protective group of hydroxyl group, it can be removed by a per se conventional means. More specifically, when a silyl group such as trimethylsilyl, tert-butyldimethylsilyl, tert-butyldiphenylsilyl, is used as the protective group, the removal can be performed by bringing the silyl group into contact with a compound containing fluorine ion (e.g. potassium fluoride, sodium fluoride, tetra-n-butylammonium fluoride), The reaction is conducted in a solvent such as tetrahydrofuran, dioxane, methanol and ethanol, optionally in the coexistence of water. The amount of the compound containing fluorine ion is about 1 ~ 10 times as much moles, preferably about 1 ~ 2 times as much moles. The reaction temperature and time are respectively about -20 °C ~ 100 °C, preferably about -5 °C ~ 30 °C and about 0.1 ~ 24 hours, preferably about 0.1 ~ 10 hours. The removal of these silyl groups can be performed bringing them into contact with a mineral acid such as hydrochloric acid, sulfuric acid, or an organic acid such as methanesulfonic acid, toluenesulfonic acid. The reaction is conducted, in most cases, in the coexistence of water, and, as the solvent, use is made of ethanol, methanol, tetrahydrofuran, dioxane or acetone which is miscible with water. In most cases, use of the acid in a catalytic amount is sufficient for the purpose, but optionally in excess amount. The reaction temperature is about -5 °C ~ 100 °C, preferably about 0 °C ~ 30 °C, and the reaction time is about 0.1 ~ 24 hours, preferably about 0.1 ~ 2 hours. Also in case of using, as the protective group, an ether-containing compound such as methoxymethyl, methoxyethoxy, it can be removed bringing into contact with an acid as mentioned above. In case of employing methyl or benzyl as the protecting group, it can be eliminated by bringing into contact with iodotrimethylsilane. The amount of iodotrimethylsilane is about 1 ~ 10 times as much moles, preferably about 1 ~ 3 times as much moles. The reaction is conducted in a solvent, as exemplified by chloroform, dichloromethane, ethyl acetate, tetrahydrofuran, dioxane, acetone. The reaction temperature is about -50 °C ~ 100 °C, preferably about -20 °C ~ 30 °C, and the reaction time is about 0.1 ~ 24 hours, prederably about 0.1 ~ 10 hours.

The following is a method of preparing compounds of the general formula [1]wherein $R^3$ and/or $R^4$ are as defined above

Compound [52], which is a compound of the general formula [1] wherein $R^3$ is OCOO-$C_{1-4}$ alkyl, and Compound [53], which is a compound of the general formula [1] wherein $R^3$ is $OCOR^{14}$ and/or $R^4$ is $C_{1-4}$ alkanoyloxy, can be prepared by, for example, subjecting compound [46] to a treatment similar to the aforementioned conversion reaction of Compound [5] → Compound [6].

Compound [52'], which is a compound of the general formula [1] wherein $R^3$ and/or $R^4$ are/is OCOS-$C_{1-4}$ alkyl substituted with ω-$C_{2-4}$ alkanoylamino can be prepared, for example by reacting a compound of the general formula [1] wherein $R^3$ is a radical of the formula -$OCOR^{13}$ wherein $R^{13}$ is $CHC\ell_2$, $C_6C\ell_5$ or 2, 4, 5-$C_6H_2C\ell_3$ with HS-$C_{1-4}$ alkyl substituted with ω-$C_{2-4}$ alkanoylamino or a salt thereof. The preferred salts are sodium salts. The reaction is preferably conducted in an organic solvent such as methanol, ethanol, tetrahydrofuran, N,N-dimethylformamide, N,N-dimethylacetoamide, dimethyl sulfoxide, or acetonitrile. The amount of thiol or salt thereof to be used is preferably about 1 to 5 moler equivalent, the reaction temperature is about 0 °C to 50 °C and the reaction time is about 1 minute to about 10 hours

Compound [54], which is a compound of the general formula [1] wherein $R^3$ is

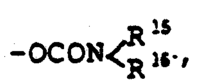

can be prepared by, for example, allowing isocyanate representable by the general formula OCN-$R^{15}$ to react with Compound [46]. The reaction is conducted preferably in an organic solvent exemplfied by dichloromethane, chloroform, 1,2-dichloroethane, tetrahydrofuran, 1,4-dioxane, acetonitrile, N,N-dimethylformamide, N,N-dimethylacetamide. The amount of isocyanate to be employed is preferably about 1 ~ 10 molar equivalent, the reaction temperature is about 0 °C ~ 150 °C and the reaction time is about 10 minutes

~ 48 hours. When about 0.1 ~ 5 molar equivalent of, for example, zinc chloride, zinc bromide, zinc iodide, cuprous chloride or di-n-butyltin dilaurate is allowed to coexist in the reaction system, the reaction temperature can be lowered by the aid of catalytic action thereof to shorten the reaction time.

Compound [54] can also be prepared by reacting a compound of the general formula [1], wherein $R^3$ is a radical -OCOR$^{13'}$ wherein $R^{13'}$ is $C_6C\ell_5$ or 2,4,5-$C_6H_2C\ell_3$ with amines represented by the general formula $HNR^{15}R^{16}$. The reaction is preferbly conducted in an organic solvent such as dichloromethane, chloroform, 1,2-dichloroethane, tetrahydrofuran, 1,4-dioxane, acetonitrile. Water may be allowed to coexist in the solvent. The amount of amine is preferably about 1 to 10 molar equivalent. The reaction temperature and the reaction time is variable according to the amine used and is about 0 °C to 100 °C and about 1 minute to about 168 hours, respectively.

As starting materials employable in the method of this invention are mentioned those described on, for example, USP 3,625,055 and they can be prepared by methods similar to those described on the literature references cited above.

Thus obtained object compounds of the invention can be isolated and refined by per se conventional means such as concentration, solvent-extraction, chromatography, crystallization, recrystallisation.

Compound [1] of this invention, when having an acid group such as carboxylic acid, sulfonic acid, phosphoric acid, etc. at its $R^1$ ~ $R^4$ portion, may form a salt by the action with a base. As the base are mentioned inorganic bases e.g. sodium, potassium, lithium, calcium, magnesium, ammonia, and organic bases e.g. pyridine, collidine, triethylamine, triethanolamine.

And, in case of having a basic group such as amino group, substituted amino group, at the $R^1$~$R^4$ portion The compounds of the invention may be in the form of an acid addition salt. As such acid addition salts are mentioned hydrochloride, hydrobromide, hydroiodide, nitrate,sulfate, phosphate, benzoate, maleate, fumarate, succinate, tartrate, citrate, oxalate, glyoxylate, asparaginate, methanesulfonate, 1,2-ethanedisulfonate, benzenesulfonate.

When compounds of the invention are obtained in the free form, they may be led to a salt by conventional means, or, conversely, that obtained in the form of a salt may be led to the free form by conventional means.

Compounds of the invention depending on cases, may form an internal salt, and these cases are also included in the present invention.

Steric isomers of compounds of the invention can be independently or as a suitable admixture of them used as anti-microbial agents.

Thus obtained compounds of the invention show strong antibacterial actions against gram positive bacteria and also against some of gram negative ones . The compounds show strong antibacterial actions against macrolide-resistant Staphylococcus aureus and meticillin cephem-resistant Staphylococcus aureus (MRSA), is well absorbed into digestive tracts and is stable in living body. The compound of the invention has antibacterial activity against Mycoplasma. Besides, toxicity of the compounds of the invention is low.

As described heretofore, the compounds of the invention have excellent antimicrobial activities and their toxicity is low. Therefore, they can be used as an antibacterial agents for the therapy of microbism of animals (e.g. domestic fowl, sheep, dog, cat, rabbit, cow, horse, monkey, man) and the therapy of animals infected with Micoplasma, and, besides, can be used as additives to feedstuff for the purpose of preventing animals from being infected with microorganisms or of promoting their growth.

The daily dosage of compounds of the invention or salts thereof varies with administration methods, species of animals to which it is to be administered and purposes of administration, but it is usually about 0.01 ~ 1000 mg/kg, more preferably about 0.1~300 mg/kg in terms of the compounds.

The compounds of the invention or a pharmaceutically acceptable salt thereof can be administered orally as, for example, tablets, granules, capsules, drops prepared by mixing with a suitable pharmcologically acceptable carrier, excipient and diluent by a conventional means, or non-orally as injections prepared by incoporating into a sterile carrier. In producing the above-described oral pharmaceutical preparations such as tablets, there can suitable be formulated binding agents (e.g., hydroxylpropyl cellulose, hydroxylpropylmethyl cellulose, Macrogol®, disintegrating agents (e.g., starch, carboxymethylcellulose calcium), excipients (e.g., lactose, starch), lubricants (e.g., magnesium stearate, talc) In manufacturing non-oral or parenteral pharmaceutical preparations, such as injectable solutions, there can suitably be formulated isotonizing agents (e.g. glucose, D-sorbitol, D-mannitol, sodium chloride), preservatives (e.g., benzyl alcohol, chlorobutanol, methyl p-oxybenzoate, propyl p-oxybenzoate), buffering agent (e.g. phosphate buffers, sodium acetate buffer)

## EXAMPLES

The following reference examples and working examples illustrate the present invention more specifically.

The abbreviations used in Tables 1 to 9 in Examples indicate by the following manner:

Cy : 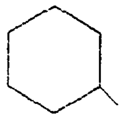 ThMe: 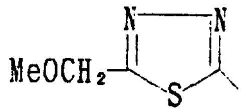

Pyn: 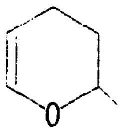

Pipe: 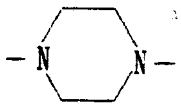 ThPe: 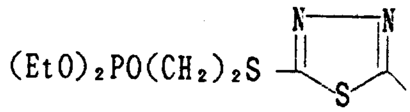

2 − Py: 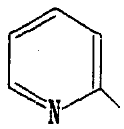 ThSm: 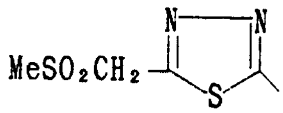

3 − Py: 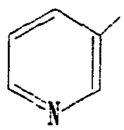 ThOh: 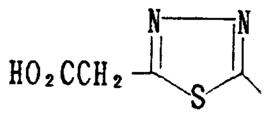

4 − Py: 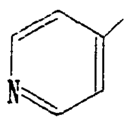 Ph: 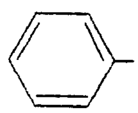

Piri: 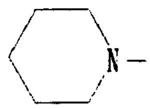

Me: $CH_3-$

Mor: 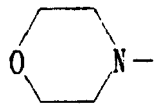

Et: $CH_3CH_2-$

Pr: $CH_3(CH_2)_2-$

iPr: $(CH_3)_2CH-$

Bu: $CH_3(CH_2)_3-$

tBu: $(CH_3)_3C-$

Pyr: 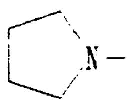

Ac: $CH_3CO-$

TeEn: 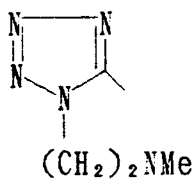

TeEO 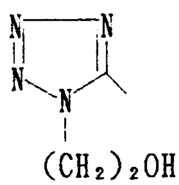

TeM 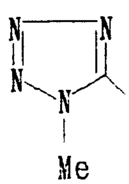

L-Ala: 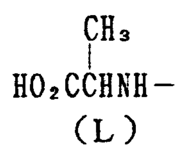

TePn 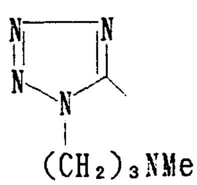

All NMR's shown in the Example, unless otherwise specified, were mesured by 90MHz in $CDC\ell_3$. The note of "DM" shows that NMR is mesured in DMSO-$d^6$ in addition to $CDC\ell_3$. "N.A." indicates uncertain proton among the signals. IR's were mesured by use of KBr and shown by $cm^{-1}$.

EXAMPLES

The following reference examples and working examples illustrate the present invention more specifically.

Reference Example 1 Preparation of lankacidin C 8,14-diacetate:

In 50 mℓ of pyridine was dissolved 5.0 g of lankacidin A. To the solution was added 25 mℓ of acetic anhydride. The mixture was stirred at room temperature for two hours. The resultant was left standing overnight at room temperature, to which was added 600 mℓ of ice-water. Precipitates then formed were collected by filtration and dissolved in ether. The ether layer was separated and dried over $MgSO_4$, from which ether was distilled off. The residue was cooled to yield crystals. The crystals were collected by filtration, washed with ether, and then dried to give 3.418 g of the above-titled compound.

NMR (90 MHz, $CDCl_3$) δ : 1.30 (d, 3H, J = 8HZ), 1.37 (s, 3H), 1.54 (s, 3H), 1.89 (s, 3H), 2.01 (s, 3H), 2.04 (s, 3H), 2.16~2.60 (m, 5H), 2.44 (s, 3H), 4.40 (dt, 1H, J = 12Hz & 3Hz), 4.70 (d, 1H, J = 14Hz), 5.05 (q, 1H, J = 8Hz), 5.20~5.87 (m, 6H), 6.26 (d, 1H, J = 14Hz), 8.06 (d, 1H, J = 10Hz).

IR (KBr): 3410, 1735, 1730, 1710, 1685, 1235cm$^{-1}$.

Reference Example 2 Preparation of 3-(2-hydroxypropionamido)-lankone 8,14-diacetate (lankacidinol 8,14-diacetate):

In 250 mℓ of methanol was dissolved 5.5 g of lankacidin C-8,14-diacetate and the solution was cooled with ice-water, to which was added dropwise a solution of 120 mg of sodium borohydride in 15 mℓ of methanol, followed by stirring for 30 minutes at the same temperature. To the mixture was added 1 mℓ of acetic acid, then methanol was distilled off to leave crystals. The crystals were dissolved in ethyl acetate (ca. 150 mℓ), washed with water, then dried. The solvent was distilled off, and the residue was subjected to a column chromatography with 250 g of silica gel using ethyl acetate - benzene (2:1) as solvent to give 1.5827 g of the title compound (an isomer of a larger Rf value, assumed as 2'-L-compound), 1.7803 g of the title compound (an isomer showing a smaller Rf value, assumed as 2'-D-compound) and 1.7344 g of the above-titled compound (2'-DL-compound).

2'-(L)-compound :

NMR (90 MHz, $CDCl_3$) δ : 1.30 (d, 3H, J = 7Hz), 1.37 (s, 3H), 1.40 (d, 3H, J = 7Hz), 1.54 (s, 3H), 1.88 (s, 3H), 2.02 (s, 3H), 2.04 (s, 3H), 2.17~2.60 (m, 5H), 3.67 (s, 1H), 4.21 (q, 1H, J = 7Hz), 4.28~4.56 (m, 1H), 4.72 (d, 1H, J = 10Hz) 5.05 (q, 1H, J = 7Hz), 5.20~5.90 (m, 6H), 6.29 (d, 1H, J = 14Hz), 7.66 (d, 1H, J = 10Hz).

IR (KBr): 3380, 1750, 1730, 1715, 1680, 1250cm$^{-1}$

2'-(D)-compound :

NMR (90 MHz, $CDCl_3$) δ : 1.24~1.49 (m, 9H), 1.55 (s, 3H), 1.88 (s, 3H), 2.03 (s, 3H), 2.05 (s, 3H), 2.16~2.60 (m, 5H), 3.70 (s, 1H), 4.10~4.55 (m, 2H), 4.70 (d, 1H, J = 10Hz), 5.03 (q, 1H, J = 7Hz), 5.22~5.88 (m, 6H), 6.30 (d, 1H, J = 14Hz), 7.66 (d, 1H, J = 10Hz).

IR (KBr): 3400, 1740, 1725, 1715, 1670, 1245cm$^{-1}$.

Reference Example 3 Preparation of 3-(2-hydroxypropionamido)-lankone 14-acetate (lankacidinol 14-acetate):

In 500 mℓ of methanol was dissolved 20.0 g of lankacidin A. The solution was cooled with ice-water, to which was added dropwise with stirring 600 mg of sodium borohydride dissolved in 60 mℓ of methanol, followed by stirring for 40 minutes. To the mixture was added 2 mℓ of acetic acid, then methanol was distilled off to leave crystals, which were dissolved in ethyl acetate - tetrahydrofuran (2:1, ca. 600 mℓ), then washed with water, and then dried over $MgSO_4$. The solvent was evaporated to give crystals. To the crystals was added a mixture of ethyl acetate and ether (1:2), and the crystals were collected by filtration, washed with the same solvent mixture, and dried to give 19.4 g of the title compound, which was an about 1:1 mixture of the isomers at 2'-position.

NMR (90 MHz, $CDCl_3$ - DMSO - $d_6$) δ : 1.25~1.50 (m, 9H), 1.53 (s, 3H), 1.87 (s, 3H), 2.02 (s, 3H), 2.2~2.6 (m, 5H), 3.8~4.3 (m, 2H), 4.45 (m, 1H), 4.69 (m, 1H), 5.25~5.85 (m, 6H), 6.30 (d, 1H, J = 14Hz), 7.82 & 7.87 (each d, 1H , J = 10Hz),

IR (KBr) : 1740, 1720 (sh.), 1706, 1634, 1234, 1010, 956cm$^{-1}$.

Reference Example 4 Preparation of 3-(2-acetoxypropionamido)-lankone 8,14-diacetate (lankacidinol 2',8,14-triacetate):

In 140 mℓ of pyridine was dissolved 19.3 g of 3-(2-hydroxypropionamido)-lankone 14-acetate. To the solution was added 70 mℓ of acetic anhydride. The mixture was stirred for 4 hours and then left standing

overnight at room temperature. The reaction mixture was poured into ice-water (ca. 1.3ℓ) and precipitates were collected by filtration. The precipitates were dissolved in ethyl acetate - tetrahydrofuran (small volume). The aqueous layer was discarded,the organic layer was dried over $MgSO_4$ and concentrated. To the concentrate was added ether, and the vessel was scratched to cause crystallisation, to which was added a mixture of ether - hexane (1:1, ca.200 mℓ). The crystals were collected by filtration, washed with the same solvent system and dried to obtain 18.3 g of the title compound, which was an about 1:1 mixture of the isomers at 2'-position. NMR, IR and TLC data of this product were in agreement with those of the mixtures obtained in the following Reference Example 5 and 6.

Reference Example 5 Preparation of 3-(2-(L)-acetoxypropionamido)-lankone 8,14-diacetate(lankacidinol 2',8,14-triacetate):

In 10 mℓ of pyridine was dissolved 1.019 g of 3-(2-(L)-hydroxy-propionamido)-lankone 8,14-diacetate (the isomer showing a larger Rf value). To the solution was added 5 mℓ of acetic anhydride, and the mixture was stirred for 2.5 hours. The mixture was left standing overnight at room temperature and then poured into ice-water (ca. 80 mℓ). The resulting precipitate was collected by filtration and re-dissolved in ethyl acetate - ether. The aqueous layer was discarded and the organic layer was dried over $MgSO_4$. The solvent was distilled off to leave crystals, to which was added ether, followed by collecting by filtration and drying to obtain 950.1 mg of the above-titled compound, m.p.209-211 °C (decomp.).

NMR (90 MHz, $CDCl_3$) δ : 1.30 (d, J = 7Hz, 17-Me), 1.42 (s, 2-Me), 1.46 (d, J = 7Hz, 2'-Me), 1.54 (s, 11-Me), 1.88 (s, 5-Me), 2.02 & 2.04 (each s, 8-OAc, 14-OAc), 2.1~2.7 (m, 9-$H_2$, 15-$H_2$, 17-H), 2.19 (s, 2'-OAc), 4.42 (m, 16-H), 4.67 (d, J = 11Hz, 4-H), 4.9~5.9 (m, 2'-H, 8-H, 3-H, 6-H, 7-H, 10-H, 13-H, 14-H), 6.27 (d, J = 15Hz, 12-H), 7.36 (d, J = 10 Hz, NH).

IR (KBr): 3430, 1730, 1705, 1675, 1500, 1368, 1240, 1022cm$^{-1}$.

Reference Example 6 Preparation of 3-(2-(D)-acetoxypropionamido)-lankone 8,14-diacetate :

Using 0.995 g of 3-(2-(D)-hydroxypropionamido)-lankone 8, 14-diacetate (the isomer showing a smaller Rf value) obtained in Reference Example 2, similar process to that of Reference Example 5 was followed to give 745.1 mg of the above-titled compound, m.p.163-165 °C.

NMR (90 MHz, $CDCl_3$) δ : 1.30 (d, J = 7Hz, 17-Me), 1.33 (s, 2-Me), 1.40 (d, J = 7Hz, 2'-Me), 1.53 (s, 11-Me), 1.86 (s, 5-Me), 2.00 & 2.03 (each s, 8-OAc, 14-OAc), 2.1~2.7 (m, 9-$H_2$, 15-$H_2$, 17-H), 2.23 (s, 2'-OAc), 4.37 (m, 16-H), 4.67 (d, J = 11Hz, 4-H), 4.9~5.9 (m, 2'-H, 8-H, 3-H, 6-H, 7-H, 10-H, 13-H, 14-H), 6.28 (d, J = 15Hz, 12-H), 7.18 (d, J = 10Hz, NH).

IR (KBr): 3430, 1730, 1706, 1684, 1506, 1366, 1240, 1020, 960cm$^{-1}$.

Reference Example 7 Preparation of lankacidin C 8-benzyl carbonate

In 4.5 mℓ of pyridine was dissolved 459 mg of lankacidin C. The solution was cooled with ice-water, to which was added while stirring 0.343 mℓ of carbobenzoxychloride. The stirring was continued for 30 minutes at the same temperature, then for 1.5 hour at room temperature. The resultant was left standing overnight at room temperature, to which was added ice-water (ca. 50 mℓ), followed by extraction with ethyl acetate. The ethyl acetate layer was washed with IN HCℓ and water in sequence, then dried over $MgSO_4$. The solvent was distilled off, and the residue was subjected to a silica-gel (50 g) column chromatography. Elution was conducted with ethyl acetate - chloroform (1:1), and the eluate was fractionated by 10 g each. The 12th ~ the 18th fractions were combined and concentrated to give 71.65 mg of the above-titled compound as an oily product, which was left standing in a freezer to become crystals, m.p.185-187 °C (decomp.)

NMR (90 MHz, $CDCl_3$) δ : 1.29 (d, J = 7Hz, 17-Me), 1.35 (s, 2-Me), 1.52 (s, 11-Me), 1.88 (s, 5-Me), 2.1~2.7 (m, 9-$H_2$, 15-$H_2$, 17-H), 2.43 (s, $COCOCH_3$), 4.07 (m, 8-H), 4.41 (m, 16-H), 4.66 (d, J = 11Hz, 4-H), 5.11 (s, $C_6H_5CH_2$), 5.1~5.8 (m, 3-H, 6-H, 7-H, 10-H, 13-H, 14-H), 6.30 (d, J = 15Hz, 12-H), 7.32 (s, $C_6H_5$), 8.05 (d, J = 10Hz, NH).

IR (KBr): 1738, 1708, 1680, 1500, 1314, 1252, 1136, 962cm$^{-1}$.

$[\alpha]_D^{26}$ - 161.8 ° (c = 0.555, $CHCl_3$)

Reference Example 8 Preparation of lankacidin C 8-benzyl carbonate

The 22nd ~ the 33rd fractions of the column chromatography conducted in Reference Example 7 were combined and concentrated to obtain 65.15 mg of the title compound as an oily product. which was left standing in a freezer to become crystals, m.p.182-184°C (decomp.).

NMR (90 MHz, $CDCl_3$) δ : 1.24 (d, J = 7Hz, 17-Me), 1.36 (s, 2-Me), 1.54 (s, 11-Me), 1.88 (s, 5-Me), 2.0~2.7 (m, 9-$H_2$, 15-$H_2$, 17-H), 2.44 (s, $COCOCH_3$), 4.2~5.1 (m, 16-H, 4-H, 13-H, 8-H, 14-H), 5.11 (s, $C_6H_5CH_2$), 5.1~6.0 (m, 3-H, 6-H, 7-H, 10-H), 6.14 (d, J = 15Hz, 12-H), 7.33 (s, $C_6H_5$), 8.06 (d, J = 10Hz, NH).

IR (KBr): 1740, 1706, 1682, 1380, 1354, 1256, 1162, 1000, 960cm$^{-1}$.

$[\alpha]_D^{26}$ - 140.9° (c = 0.46, $CHCl_3$)

Reference Example 9 Preparation of lankacidin C 8,14-bischloroacetate:

In 10 mℓ of dichloromethane was suspended 459 mg of lankacidin C. To the suspension were added 366 mg of 4-dimethylaminopyridine and 513 mg of monochloroacetic anhydride. The mixture was stirred for 8.5 hours at room temperautre, followed by being left standing overnight. The resultant was washed with water, then dried over $MgSO_4$. The dichloromethane was evaporated to leave crystals. To the crystals was added ether, and the insoluble crystals were filtered off. The filtrate was concentrated. The concentrate was purified by means of preparative TLC using TLC-plates (manufactured by Merck, Art. No.5715, 20 x 20 cm, 2 plates, developing solvent: ethyl acetate - hexane (2:1)) to give 3.9 mg of the above-titled compound as a colorless oily product.

NMR (90 MHz, $CDCl_3$) δ : 1.31 (d, J = 7Hz, 17-Me), 1.38 (s, 2-Me), 1.53 (s, 11-Me), 1.90 (s, 5-Me), 2.2~2.7 (m, 9-$H_2$, 15-$H_2$, 17-H), 2.45 (s, $COCOCH_3$), 4.01 & 4.03 (each s, $ClCH_2$ × 2), 4,43 (m, 16-H), 4.72 (d, J = 10Hz, 4-H), 5.0~6.1 (m, 8-H, 3-H, 6-H, 7-H, 10-H, 13-H, 14-H), 6.32 (d, J = 15Hz, 12-H), 8.05 (d, J = 10Hz, NH).

IR (KBr): 1740, 1704, 1680, 1252, 1160, 960cm$^{-1}$.

Reference Example 10 Preparation of lankacidin A 8-benzoate :

In 5 mℓ of pyridine was dissolved 501 mg of lankacidin A. To the solution was added, while stirring under cooling with ice-water, dropwise 0.174 mℓ of benzoyl chloride. Five minutes later, the ice-water bath was removed, and the mixture was stirred at room temperature. Thirty-five minutes later, 0.087 mℓ of benzoyl chloride was further added, followed by stirring for further 30 minutes. The resultant was poured into ice-water and the resulting precipitate was extracted with ethyl acetate. The ethyl acetate layer was washed with 1 N HCℓ and an aqueous NaCl solution in sequence, followed by dring over $MgSO_4$. The solvent was distilled off to leave crystals, to which was added a mixture of ether and petroleum ether (1:1). The crystals were collected by filtration and washed with the same solvent and then dried to give 476.8 mg of the above-titled compound, m.p.221-223°C (decomp.).

NMR (90 MHz, $CDCl_3$) δ : 1.32 (d, J = 7Hz, 17-Me), 1.38 (s, 2-Me), 1.59 (s, 11-Me), 1.92 (s, 5-Me), 2.02 (s, OAc), 2.2~2.7 (m, 9-$H_2$, 15-$H_2$ 17-H), 2.43 (s, $COCOCH_3$), 4.32 (m, 16-H), 4.73 (d, J = 11Hz, 4-H), 5.2~5.9 (m, 8-H, 3-H, 6-H, 7-H, 10-H, 13-H, 14-H, 6.32 (d, J = 15Hz, 12-H), ~7.5 (m, 3H, $C_6H_5$), ~ 8.1 (m, 3H, $C_6H_5$, NH).

IR (KBr): 1708, 1356, 1270, 1240, 1112, 952cm$^{-1}$.

$[\alpha]_D^{25}$ - 124.2° (c = 0.48, $CHCl_3$)

Reference Example 11 Preparation of the esterase for 14-O-deacylation

In a 40ℓ vessel was put 5ℓ of the culture filtrate of Streptomyces rochei var. volubilis No. T-2636 (IFO 12507, FERM P-6155) [the culture filtrate described in the working example disclosed on an official gazette of Toku Ko Sho 47-20959], to which was added 20ℓ of 95% ethanol. The mixture was stirred sufficiently and then left standing for 12 hours at 10°C or below. The supernatant was removed by means of a siphon to leave a slurry consisting of white precipitates and liberated products. This slurry was subjected to centrifuge (5~10°C, 2000 x g or more). The soil-like substance thus obtained was dried in vacuo at 10°C or below for one full day (50 µHg or below) to give a greyish white enzyme preparation.

Reference Example 12 Preparation of lankacidin C :

In 20 mℓ of methanol was dissolved 102 mg of lankacidin A. To the solution was added a 80 mℓ aqueous solution of 2.0 g of the enzyme prepared in Reference Example 11. The mixture was stirred at room temperature for 35 minutes, then methanol was distilled off. The residue was extracted with methyl isobutyl ketone - tetrahydrofuran (1:1). The extract was dried on $MgSO_4$, then the solvent was distilled off. To the residue was added ether to give a white powdery crystalline substance, which was collected by filtration and dried to obtain 24.85 mg of the title compound. This product was in agreement with lankacidin C obtained by a fermentative method in NMR, IR and TLC.

Referecne Example 13 Preparation of lankacidin C-8-acetate :

In a mixture of 20 mℓ of methanol and 10 mℓ of tetrahydrofuran was dissolved in 109 mg of lankacidin C 8,14-diacetate. To the solution was added 80 mℓ of an aqueous solution of 2.0 g of the enzyme prepared in Reference Example 11, and the mixture was stirred at room temperature for 50 minutes. Methanol and tetrahydrofuran were distilled off, and the residue was extracted with chloroform - acetone. The organic layer was dried over $MgSO_4$, then the solvent was distilled off. The residue was subjected to a silica gel (60 g) column chromatography, then elution was conducted with ethyl acetate - chloroform (2:1), and the eluate was fractionated by 8 g each. The 25th~the 36th fractions were combined and the solvent distilled off to yield 42.2 mg of the title compound as white crystals

NMR (90 MHz, $CDCl_3$) $\delta$ : 1.28 (d, J = 7Hz, 17-Me), 1.37 (s, 2-Me), 1.55 (s, 11-Me), 1.90 (s, 5-Me), 2.03 (s, OAc), 2.2~2.6 (m, 9-$H_2$, 15-$H_2$, 17-H), 2.43 (s, $COCOCH_3$), 4.33 (m, 14-H), 4.43 (m, 16-H), 4.70 (d, J = 11Hz, 4-H), 5.05 (m, 8-H), 5.2~5.9 (m, 3-H, 6-H, 7-H, 10-H, 13-H), 6.15 (d, J = 15Hz, 12-H), 8.07 (d, J = 10Hz, NH).

IR (KBr): 3475, 1728, 1706, 1672, 1256, 1234 (sh.), 1012, 960$cm^{-1}$.

Reference Example 14 Preparation of lankacidin C :

In a mixture of 10 mℓ of dichloromethane and 1 mℓ of acetic acid was dissolved 405 mg of lankacidin C 8,14-bis (2,2,2-trichloroethylcarbonate). To the solution was added 810 mg of zinc powder, and the mixture was stirred at room temperature for 5 hours. To the resultant was added ethyl acetate, then insolubles were filtered off by using filter aid, and the filtrate was concentrated. The concentrate was subjected to a silica gel (50 g) column chromatography, elution being conducted with a mixture of tetrahydrofuran and chloroform (1:2). The eluate was fractionated by 10 g each. The 20th~the 28th fractions were combined, from which was distilled off the solvent. To the residue was added ether, and the wall of the vessel was scratched to yield 45.5 mg of the above-titled compound. This product was in agreement with the lakacidin C obtained by a fermentative process in NMR, IR, and TLC.

Reference Example 15 Preparation of lankacidin C :

In 1 mℓ of tetrahydrofuran was dissolved 60.4 mg of lankacidin C 8,14-bistrimethylsilylether. To the solution was added 0.3 mℓ of 1N HCℓ, and the mixture was stirred for 10 minutes at room temperature. To the resultant was added ethyl acetate, which was washed with water, an aqueous solution of sodium hydrogencarbonate and an aqueous saline solution in sequence, then dried over $Na_2SO_4$, followed by distilling off the solvent to leave 50.5 mg of the above-titled compound. This product was in agreement with the lankacidin C obtained by a fermentative process in NMR, IR and TLC.

Reference Example 16 Preparation of lankacidin C :

In 0.5 mℓ of tetrahydrofuran was dissolved 20.6 mg of lankacidin C 8,14-bis(dimethyl-t-butylsilylether). To the solution was added 0.1 mℓ of 2N HCℓ , and the mixture was stirred at room temperature for 2.5 hours. To the resultant was added ethyl acetate, which was washed with water, an aqueous solution of sodium hydrogencarbonate and an aqueous saline solution in sequence, then dried over $Na_2SO_4$, followed by distilling off the solvent. The residue was purified by means of preparative TLC using TLC-plates (manufactured by Merk, Art. No.5715, 20 x 20 cm, developing solvent : ethyl acetate) to obtain 17.4 mg of the above-titled compound. This product was in agreement with the lankacidin C obtained by a fermentative process in NMR, IR AND TLC.

Reference Example 17 Preparation of 8-dehydroxy-8-chloro-lankacidin A :

In 5 mℓ of dichloromethane was dissolved 501 mg of lankacidin A. To the solution was added 89μℓ of pyridine, and the mixture was cooled at 0°C. To the resultant mixture was added dropwise 80.2μℓ of thionyl chloride. The mixture was stirred at the same temperature for 30 minutes, to which was added ice-water, followed by extraction with dichloromethane. The extract was dried over $MgSO_4$ and the solvent was distilled off. The residue was subjected to a silica gel (50 g) column chromatography with ethyl acetate - chroform (1:4). The eluate was fractionated by 15 g each. The 11th to the 23rd fractions were combined and the solvent was distilled off. To the residue was added ether, whereupon crystals separated, to which was added ether - petroleum ether (1:2). The crystals were collected by filtration and then dried to obtain 339.9 mg of the title compound as crystals.

NMR (90 MHz, $CDCl_3$) δ : 1.30 (d, J = 7Hz, 17-Me), 1.37 (s, 2-Me), 1.56 (s, 11-Me), 1.91 (s, 5-Me), 2.02 (s, OAc), 2.1~2.8 (m, 15-$H_2$, 17-H, 9-$H_2$), 2.45 (s, $COCOCH_3$), 4.14 (m, 8-H), 4.42 (m, 16-H), 4.71 (d, J = 11Hz, 4-H), 5.2~5.9 (m, 3-H, 6-H, 10-H, 13-H, 14-H), 6.26 (d, J = 15Hz, 12-H), 8.06 (d, J = 10 Hz, NH).

IR (KBr): 3380, 1740, 1708, 1700 (sh.), 1506, 1356, 1256, 1224, 1138, 946$cm^{-1}$.

Mass m/e: 519($M^+$), 483($M^+$ - 36(HCl)), 459($M^+$ - 60(AcOH)), 423($M^+$ - 36 - 60)

Reference Example 18 Preparation of bis[3-(2-(D)-hydroxy-propinonamido)-lankone 8.14-diacetate-2'(O)-yl]-sulfone :

In 0.5 mℓ of pyridine was dissolved 86.7 mg of 3-(2-(D)-hydroxypropionamido)-lankone 8.14-diacetate. To the solution was added 12.7μℓ of thionyl chloride under cooling with ice-water, and the mixture was stirred for 20 minutes. To the resultant was added ice-water and the precipitates formed were collected by filtration, followed by recrystallization from ethyl acetate - ether to give 60.1 mg of the above-titled compound, m.p.182-183°C.

NMR (90 MHz, $CDCl_3$) δ : 1.20~1.67 (m, 12H), 1.87 (s, 3H), 2.02 (s, 3H), 2.04 (s, 3H), 2.15~2.60 (m, 5H), 4.60~5.90 (m, 10H), 6.28 (d, 1H, J = 14Hz), 7.40~7.70 (m, 1H).

IR (KBr): 3420, 1730, 1715, 1690, 1245$cm^{-1}$.

Reference Example 19 Preparation of 2-(2,2,2-trichloroethoxycarbonyl)phenylacetyl chloride :

1) In 80 mℓ of dichloromethane was suspended 5.0 g of phenylmalonic acid. To the suspension was added 4.25 g of 2,2,2-trichloroethanol. To the mixture was added little by little 5.8 g of dicyclohexylcarbodiimide under ice-cooling while stirring. The stirring was continued at the same temperature for 20 minutes, then at room temperature for further 3 hours. The precipitates formed were then filtered off, and the precipitates were washed with a small volume of dichloromethane. The filtrate was washed with water and extracted with an aqueous solution of sodium hydrogencarbonate. The aqueous layer was acidified with 1N HCℓ, then extracted with ethyl acetate. The extract was washed with saline, dried over $MgSO_4$ and the solvent was distilled off. To the residue was added chloroform and some precipitated phenyl malonic acid was removed by filtration. The filtrate was concentrated to give 3.2 g of crude phenyl malonic acid mono(2,2,2-trichloroethyl)ester.

NMR (60 MHz, $CDCl_3$) δ : 4.78 (s, 3H), 7.37 (s, 5H), 9.5 (br.,1H).

2) In 12 mℓ of thionyl chloride was dissolved 3.2 g of the above-mentioned phenyl malonic acid mono-(2,2,2-trichloroethyl)ester. The solution was stirred at 50°C for one hour, which was then refluxed for 30 minutes. The reaction solution was concentrated, followed by removing distillate by means of a vacuum pump (bath temperature : 140°C) to obtain 2.4 g of the above-titled compound as an orange oily product.

NMR (60 MHz, $CDCl_3$) δ : 4.80 (s, 2H), 5.13 (s, H), 7.40 (s, 5H).

Reference Example 20 Preparation of D(-)-2-(2,2,2-trichloroethoxycarbonylamino)phenylacetyl chloride :

In 12 mℓ of dichloromethane was suspended 0.88 g of D(-)-2-(2,2,2-trichloroethoxycarbonylamino)-phenylacetic acid. To the suspension, while stirring under ice-cooling, were added one drop of N,N-dimethylformamide and 0.6 mℓ of oxalyl chloride. The mixture was stirred for 10 minutes at the same temperature and for 20 minutes at room temperature, followed by concentration to obtain 1.0 g of a crude product of the above-titled compound as yellowish orange oily substance.

NMR (60 MHz, $CDCl_3$) δ : 4.77 (s, 2H), 5.62 (br.d, 1H, J = 7Hz), 5.9 (br., 1H), 7.42 (s, 5H).

Example 1 Preparation of 3-(2-acetoxyacrylamido)-lankone 8, 14-diacetate :

In 1 mℓ of dichloromethane was dissolved 54.3 mg of lankacidin C 8,14-diacetate. To the solution were added 13.9 µℓ of triethylamine, 18.9 µℓ of acetic anhydride and 12.2 mg of 4-(N,N-dimethylamino)pyridine. The mixture was stirred at room temperature for 9 hours, which was left standing overnight at room temperature. To the resultant was added dichloromethane, followed by washing with 2 mℓ of 0.5N hydrochloric acid and saline in sequence then drying over $MgSO_4$. Dichloromethane was distilled off, and the residue was subjected to separation by means of preparation TLC Plates: manufactured by Merck, Art. No.5715, 20 x 20 cm, two paltes, developing solvent : a mixture of ethyl acetate and chloroform (1:4) to obtain 13.9 mg of the title compound.

NMR (90 MHz, $CDCl_3$) δ : 1.29 (d, J = 7Hz, 17-Me), 1.38 (s, 2-Me), 1.54 (s, 11-Me), 1.89 (s, 5-Me), 2.02 & 2.04 (each s, 8-OAc, 14-OAc), 2.2~2.7 (m, 9-$H_2$, 15-$H_2$, 17-H), 2.33 (s, 2'-OAc), 4.39 (m, 16-H), 4.67 (d, J = 11Hz, 4-H), 5.07 (m, 8-H), 5.2~6.2 (m, 3-H, 6-H, 7-H, 10-H, 13-H, 14-H, = $CH_2$), 6.27 (d, J = 15Hz, 12-H), 7.35 (d, J = 10Hz, NH).

IR (KBr): 1738, 1712, 1372, 1244, 1172, 1022$cm^{-1}$.

Reference Example 21 Preparation of lankacidin C 8-(2,2,2-trichloroethylcarbonate) :

In 20 mℓ of tetrahydrofuran was dissolved 135.3 mg of lankacidin A 8-(2,2,2-trichloroethylcarbonate), to which was added 20 mℓ of methanol. To the mixture was added 40 mℓ of aqueous solution containing 2.7 g of the enzyme prepared in Reference Example 11, followed by stirring at room temperature for 50 minutes. The resultant mixture was subjected to extraction with 80 mℓ of chloroform. The chloroform layer was washed with aqueous NaCl solution and was dried over $MgSO_4$. Chloroform was distilled off. To the residue was added a small volume of ether, and the mixture was left standing to cause crystallization, to which was added ether - petroleum ether (1:1). The crystals were collected by filtration, followed by drying to obtain 82.9 mg of the above titled compound, m.p.209-211 °C (decomp.).

NMR (90 MHz, $CDCl_3$) δ : 1.28 (d, J = 7Hz, 17-Me), 1.38 (s, 2-Me), 1.57 (s, 11-Me), 1.91 (s, 5- Me), 2.2~2.7 (m, 9-$H_2$, 15-$H_2$, 17-H), 2.43 (s, $COCOCH_3$), ~4.4 (m, 16-H, 14-H), ~4.7 (m, 4-H), 4.73 (s, $CCl_3CH_2$), 4.97 (m, 8-H), 5.15~5.95 (m, 3-H, 6-H, 7-H, 10-H, 13-H), 6.18 (d, J = 15Hz, 12-H), 8.07 (d, J = 10Hz, NH).

IR (KBr): 1748, 1704, 1674 (sh.), 1376, 1244, 954$cm^{-1}$.

$[\alpha]_D^{25}$ - 183.8° (c = 0.495, $CHCl_3$)

In a manner similar to the above, 14-acetate was subjected to deacetylation with an enzyme giving the corresponding 14-hydroxy-compounds of the following Examples.

Example 2 Preparation of 3-(2-oxo-1-thioxopropylamino)-lankone 8-acetate :

The yield was 94%. m.p.: 201-203 °C

NMR (90 MHz, $CDCl_3$) δ : 1.27 (d, J = 7Hz, 17-Me), 1.41 (s, 2-Me), 1.56 (s, 11-Me, 1.95 (s, 5-Me), 2.03 (s, OAc), 2.2~2.6 (m, 9-$H_2$, 15-$H_2$, 17-H), 2.63 (s, $CSCOCH_3$), 3.72 (m, 14-OH), 4.32 (m, 14-H), 4.43 (m, 16-H), 4.69 (d, J = 11Hz, 4-H), 5.04 (m, 8-H), 5.2~6.15 (m, 3-H, 6-H, 7-H, 10-H, 13-H), 6.15 (d, J = 15Hz, 12-H), 9.98 (d, J = 10Hz, NH).

IR (KBr): 3330, 1740, 1708, 1494, 1378, 1350, 1262, 1210, 1026, 964$cm^{-1}$.

$[\alpha]_D^{25}$ - 409.1° (c = 0.47, $CHCl_3$)

Example 3 3-(2-(L)-hydroxy-1-thioxopropylamino)-lankone 8-acetate :

The yield was 61%. m.p.: 213-214 °C (decomp.)

NMR (90 MHz, $CDCl_3$ - DMSO-$d_8$ (4 : 1)) δ : 1.25 (d, J = 7Hz, 17-Me), 1.37 (s, 2-Me), 1.43 (d, J = 7Hz, 2'-Me), 1.53 (s, 11-Me), 1.92 (s, 5-Me), 2.01 (s, OAc), 2.1~2.7 (m, 9-$H_2$, 15-$H_2$, 17-H), 4.1~4.6 (m, 2'-H, 14-H), ~4.5 (m, 16-H), 4.78 (d, J = 11Hz, 4-H), 4.99 (m, 8-H), 5.15~6.35 (m, 3-H, 6-H, 7-H, 10-H, 13-H), 6.13 (d, J = 15Hz, 12-H), 9.77 (d, J = 10Hz, NH).

IR (KBr): 1730, 1710, 1500, 1372, 1260, 1064, 1020, 996, 964$cm^{-1}$.

$[\alpha]_D^{25}$ - 366.0° (c = 0.52, $CHCl_3$)

Example 4 3-(2-(D)-hydroxy-1-thioxopropylamino)-lankone 8-acetate ;

The yield was 81%. m.p.: 156-158 °C (decomp.)

NMR (90 MHz, $CDCl_3$-DMSO-$d_6$ (4 : 1)) δ : 1.25 (d, J = 7Hz, 17-Me), 1.40 (d, J = 7Hz, 2'-Me), 1.41 (s,

2-Me), 1.55 (s, 11-Me), 1.93 (s, 5-Me), 2.02 (s, OAc), 2.1~2.7 (m, 9-$H_2$, 15-$H_2$, 17-H), 4.1~4.7 (m, 2'-H, 14-H, 16-H), 4.75 (d, J = 11Hz, 4-H), 5.03 (m, 8-H), 5.1~6.3 (m, 3-H, 6-H, 7-H, 10-H, 13-H), 6.14 (d, J = 15Hz, 12-H), 9.85 (d, J = 10Hz, NH).

IR (KBr): 3520, 3300, 1730, 1702, 1498, 1374, 1260 (sh.), 1248, 1064, 958cm$^{-1}$.

$[\alpha]_D^{25}$ -272.6° (c = 0.53, $CHCl_3$)

Example 5 8-Dehydroxy-8-(1-dimethylamino-1H-tetrazol-5-yl)thio-lankacidin C :

The reaction was allowed to proceed at room temperature for 5.5 hours. The yield was 78%.

NMR (90MHz, $CDCl_3$) δ : 1.27 (d, J = 6.5Hz, 17-Me), 1.38 (s, 2-Me), 1.54 (s, 11-Me), 1.90 (s, 5-Me), ~ 2.0 (br., OH), 2.2~2.85 (m, 9-$H_2$, 15-$H_2$, 17-H), 2.44 (s, $COCOCH_3$), 2.95 (s, $NMe_2$), 4.2~4.55 (m, 14-H, 16-H), 4.72 (d, J = 11Hz, 4-H), 4.93 (m, 8-H), 5.3~6.0 (m, 3-H, 6-H, 7-H, 10-H, 13-H), 6.23 (d, J = 15Hz, 12-H), 8.07 (d, J = 10Hz, NH).

IR (KBr): 3390, 2980, 2930, 1745, 1710, 1685, 1500, 1440, 1380, 1355, 1250, 1220, 1160, 1135, 1055, 1010, 960, 745cm$^{-1}$.

Reference Example 22 Preparation of Lankacidin A 8-iodomethyl carbonate :

In 120 mℓ of acetonitrile was dissolved the crude Lankacidin A 8-chloromethyl carbonate (8.2 g of Fr.1 + 5.5 g of Fr.2). To the solution was added 11.2 g of sodium iodide. The mixture was stirred at 50~60°C for 3 hours, to which was added 1.5ℓ of ethyl acetate, followed by washing with 300 mℓ of water containing 19.3 g of $Na_2S_2O_3$, 200 mℓ of water and 200 mℓ of aqueous NaCl solution in sequence and drying over $MgSO_4$. The solvent was then distilled off, and the residue was subjected to a silica gel (320 g) column chromatography, eluting with chloroform - ethyl acetate (30:1, then 20:1). The fractions containing the above titled compound was concentrated to obtain 13.8 g of yellow foamy substance, to which was added ether (ca.100 mℓ). The mixture was left standing, then crystals separated out, which were collected by filtration and dried to obtain 9.5 g of the above titled compound. This compound was found to contain about 5% of 8-chloromethyl carbonate and 8-dichloromethyl carbonate. In the subsequent working exampies, this compound was used without further purification. Melting point : 147-149°C (decomp.)

NMR (90MHz, $CDCl_3$) δ : 1.31 (3H, d, J = 6.5Hz), 1.37 (3H, s), 1.54 (3H, s), 1.90 (3H, s), 2.01 (3H, s), 2.2~2.65 (5H, m), 2.44 (3H, s), 4.41 (1H, m), 4.71 (1H, d, J = 10Hz), 4.96 (1H, m), 5.22~6.0 (6H, m), 5.91 (2H, s), 6.27 (1H, d, J = 15Hz), 8.06 (1H, d, J = 9Hz).

IR (KBr): 3400, 1760, 1680, 1510, 1360, 1280, 1235, 1220, 1160, 1135, 1070, 1020, 930cm$^{-1}$.

Reference Example 23 Preparation of lankacidin A 8-chloroacetate:

In 250 mℓ of N,N-dimethylacetamide was dissolved 20 g of lankacidin A, to which was added dropwise while stirring 5 mℓ of chloroacetyl chloride. The mixture was stirred at room temperature for 2.5 hours, to which was added 1 ℓ of ethyl acetate. The mixture was washed with water, 5% aqueous solution of sodium hydrogencarbonate, 1N HCℓ, water and saturated aqueous NaCl solution, in sequence, followed by drying over $MgSO_4$. The solvent was distilled off. To the residue was added 200 mℓ of isopropyl ether. The mixture was left standing, then crystals separated out. The crystals were collected by filtration, followed by drying to obtain 21.8 g of the above titled compound, m.p.192-198°C (decomp.).

NMR (90MHz, $CDCl_3$) δ : 1.30 (3H, d, J = 6 Hz), 1.38 (3H, s), 1.56 (3H, s), 1.90 (3H, s), 2.02 (3H, s), 2.44 (3H, s), 2.2~2.7 (5H, m), 4.03 (2H, s), 4.42 (1H, dt, J = 3 & 12Hz), 4.72 (1H, d, J = 10.5Hz), 5.0~5.9 (7H, m), 6.28 (1H, d, J = 15Hz), 8.07 (1H, d, J = 10.5Hz).

IR (KBr): 3400, 2960, 2870, 1740, 1710, 1510 cm$^{-1}$.

Reference Example 24 Preparation of lankacidin A 8-phenyl carbonate :

In 5 mℓ of pyridine was dissolved 501 mg of lankacidin A. To the solution was added dropwise 0.188 mℓ of phenyl chloroformate under ice-cooling while stirring. The mixture was stirred at the same temperature for 5 minutes, then at room temperature for 75 minutes. To the resultant was added ice-water, which was subjected to extraction with ethyl acetate. The extract solution was washed with 1N HCℓ and aqueous saline solution in sequence, which was dried on $MgSO_4$, followed by distilling off the solvent. To the residue was added ether, whereupon crystallization occurred. To this was added ether - petroleum ether (1:1), then the crystals were collected by filtration, followed by drying to obtain 473.9 mg of the above titled compound, m.p.220-222°C (decomp.).

NMR (90 MHz, $CDCl_3$) δ : 1.32 (3H, d, J = 7 Hz), 1.40 (3H, s), 1.59 (3H, s), 1.95 (3H, s), 2.04 (3H, s), 2.2~2.8 (5H, m), 2.47 (3H, s), 4.43 (1H, m), 4.75 (1H, d, J = 11Hz), 5.03 (1H, m), 5.2~6.0 (6H, m), 6.32 (1H, d, J = 15Hz), 7.1~7.55 (5H, m), 8.10 (1H, d, J = 10Hz).

IR (KBr): 1740, 1706, 1684, 1354, 1240, 1208, 952$cm^{-1}$.

In a manner similar to that of Reference Example 24, the following compounds were obtained.

Example 6 Lankacidinol A 8-methylcarbonate, m.p.215-216 ° C (decomp.)

NMR (90 MHz, $CDCl_3$) δ : 1.32 (3H, d, J = 7 Hz), 1.39 (3H, s), 1.56 (3H, s), 1.91 (3H, s), 2.04 (3H, s), 2.2~2.6 (5H, m), 3.78 (3H, s), 4.44 (1H, m), 4.74 (4H, d, J = 11Hz), 4.93 (1H, m), 5.2~5.9 (6H, m), 6.31 (1H, d, J = 15Hz), 8.09 (1H, d, J = 10Hz).

IR (KBr): 1740, 1706, 1684, 1500, 1440, 1356, 1260, 948$cm^{-1}$.

Reference Example 25 Lankacidin A 8-pentachlorophenylcarbonate, m.p.185-187 ° C (decomp.)

NMR (90 MHz, $CDCl_3$) δ : 1.31 (3H, d, J = 7Hz), 1.38 (3H, s), 1.59 (3H, s), 1.94 (3H, s), 2.03 (3H, s), 2.15~2.8 (5H, m), 2.46 (3H, s), 4.43 (1H, m), 4.75 (1H, d, J = 11Hz), 5.05 (1H, m), 5.2~5.95 (6H, m), 6.30 (1H, d, J = 15Hz), 8.10 (1H, d, J = 10Hz).

IR (KBr): 1780 (sh.), 1756, 1710, 1688, 1360, 1240$cm^{-1}$.

Reference Example 26 Preparation of of lankacidin A 8-iodoacetate :

In 300 mℓ of acetonitrile was dissolved 10 g of lankacidin A 8-chloroacetate. To the solution was added 10 g of sodium iodide, and the mixture was stirred at 55 ° C for 18 hours The acetonitrile was distilled off. To the residue was added 600 mℓ of ethyl acetate, which was washed with water, aqueous solution of sodium thiosulfate, water and saturated aqueous NaCl solution, in sequence, followed by drying on $MgSO_4$. The solvent was distilled off. To the residue was added 110 mℓ of ether, whereupon crystals precipitated out. The crystals were collected by filtration and dried to give 8.9 g of the above tiltled compound, m.p.191-197 ° C(decomp.).

NMR (90 MHz, $CDCl_3$) δ : 1.30 (3H, d, J = 6 Hz), 1.38 (3H, s), 1.57 (3H, s), 1.90 (3H, s), 2.03 (3H, s), 2.1~2.7 (5H, m), 2.46 (3H, s), 3.67 (2H, s), 4.40 (1H, dt, J = 3 & 12Hz), 4.71 (1H, d, J = 10.5Hz), 4.9~5.9 (7H, m), 6.28 (1H, d, J = 15Hz), 8.08 (1H, d, J = 10.5Hz).

IR (KBr): 3400, 2940, 1725, 1675, 1500$cm^{-1}$.

Reference Example 27 Preparation of lankacidin A 8-N-phenylcarbamate :

In 6 mℓ of dichloromethane was dissolved 300.6 mg of lankacidin A. To the solution were added 180 mg of zinc chloride and 0.131 mg of phenyl isocyanate. The mixture was stirred at room temperature for 19 hours. To the resultant mixture was added dichlromethane, and insolubles were removed by decantation. The liquid portion was concentrated and subjected to a silica gel (75 g) column chromatography, eluting with ethyl acetate - chloroform (1:10). The eluate was fractionated by 20 g each portion. The 34th ~ the 43rd fractions were combined and concentrated to leave crystals, to which was added ether, then the crystals were collected by filtration, washed with ether and dried to obtain 121.1 mg of the above titled compound as white crystals, m.p.231-232 ° C (decomp.).

NMR (90 MHz, $CDCl_3$) δ : 1.31 (3H, d, J = 7 Hz), 1.37 (3H, s), 1.55 (3H, s), 1.89 (3H, s), 2.02 (3H, s), 2.2~2.6 (5H, m), 2.44 (3H, s), 4.40 (1H, m), 4.70 (1H, d, J = 11Hz), 5.05 (1H, m), 5.25~5.9 (6H, m), 6.28 (1H, d, J = 15Hz), 6.73 (1H, br.s), 6.9~7.5 (5H, m), 8.05 (1H, d, J = 10Hz).

IR (KBr): 1724 (sh.), 1706, 1682, 1514, 1438, 1236 (sh.), 1220$cm^{-1}$.

Employing lankacidin A as the starting material, reactions were conducted in a manner similar to that of Reference Example 27 to obtain compounds shown in Table 1.

Table 1

8-position($R^3$) derivatives of Lankacidin A

| Reference Example No. | $R^3$ | Yield (%) | m.p. (°C) | NMR: 11-Me, COCOCH3, 8-H, others | IR(carbonyl region) |
|---|---|---|---|---|---|
| 28 | MeNHCOO- | 29.9 | — | 1.53(s), 2.44(s), 4.98(m), 2.77(d, J = 5Hz NHMe) | 1720(sh), 1708, 1684(sh) |

Example 7 Preparation of O(8)-morpholinocarbonyl lankacidin A :

In 6 mℓ of dichloromethane was dissolved 476.1 mg of lankacidin A 8-pentachlorophenyl carbonate. To the solution was added 0.104 mℓ of morpholine, and the mixture was stirred at room temperature for 2

hours. To the resultant mixture was added dichlorometnane and the solution was washed with water then dried on $MgSO_4$. The solvent was distilled off, and the residue was subjected to a silica gel (100 g) column chromatography, eluting with ethyl acetate - chloroform (1:4) then with ethyl acetate - chloroform (1:1). The eluate was concentrated. To the concentrate was added a small volume of ether to cause crystallization, to which was added ether - petroleum ether (1:1), followed by collecting the crystals and drying to obtain 313.0 mg of the above titled compound as white crystals, m.p.223-225 °C (decomp.).

NMR (90 MHz, $CDCl_3$) $\delta$ : 1.31 (3H, d, J = 7 Hz), 1.38 (3H, s), 1.56 (3H, s), 1.91 (3H, s), 2.03 (3H, s), 2.2~2.7 (5H, m), 2.46 (3H, s), ~ 3.6 (8H, m), 4.43 (1H, m), 4.71 (1H, d, J = 11Hz), 5.02 (1H, m), 5.25~5.9 (6H, m), 6.32 (1H, d, J = 15Hz), 8.07 (1H, d, J = 10Hz).

IR (KBr): 1750 (sh.), 1730 (sh.), 1710, 1695, 1426, 1360, 1238, 1132, 954cm$^{-1}$.

Employing lankacidin A 8-pentachlorophenyl carbonate as the starting material, reactions were conducted in a manner similar to that of Example 7 to obtain compounds shown in Table 2.

Table 2

8-position($R^3$) derivatives of Lankacidin A

| Example No. | $R^3$ | Yield (%) | m.p. (°C) | NMR: 11-Me, $COCOCH_3$, 8-H, others | IR(carbonyl region) |
|---|---|---|---|---|---|
| 8 | H-Pipe-COO- | 53.7 | — | 1.56(s), 2.46(s), 5.01 (m), ~2.3(br, piperadine-NH) | 1750(sh) |
| 9 | 2-Py-Pipe-COO- | 76.1 | 212-213 (dec) | 1.56(s), 2.44(s), 5.03 (m), 6.63, 7.50, 8.18 (each m, Py) | 1750, (sh), 1728 (sh), 1705, 1690 (sh) |
| 10 | Me-Pipe-COO- | 59.1 | 210-212 (dec) | 1.56(s), 2.46(s), 5.01 (m), 2.30(s, N-Me) | 1700(sh), 1688 |
| 11 | 4-Py-Pipe-COO- | 46.8 | 175-177 | 1.54(s), 2.43(s), 5.01 (m), 6.55-6.75 & 8.15 -8.45(Py-H) | 1740(sh), 1705, 1685 |

Reference Example 29 Preparation of lankacidin A 8-(1-methyl-1H-tetrazol-5-yl)thioacetate :

In 2 mℓ of N,N-dimethylformamide was dissolved 200 mg of 1-methyl-1H-tetrazol-5-thiol. To the solution was added 24 mg of sodium hydride (60%), which was stirred at room temperature for 10 minutes.

To the mixture was added dropwise 500 mg of lankacidin A 8-chloroacetate dissolved in 2 mℓ of N,N-dimethylformamide, taking 5 minutes. The mixture was stirred for further 30 minutes. To the resultant mixture was added 50 mℓ of ethyl acetate, and the mixture was washed with water, 1N HCℓ, water and saturated aqueous saline solution, in sequence, followed by dying over $MgSO_4$. The solvent was distilled off, and the residue was subjected to a silica gel (60 g) column chromatography, eluting with ethyl acetate - chloroform (1:4 then 1:2) to obtain 560 mg of the above titled compound, m.p.131-139°C.

NMR (90 MHz, $CDCl_3$) δ : 1.31 (3H, d, J = 6 Hz), 1.40 (3H, s), 1.57 (3H, s), 1.92 (3H, s), 2.03 (3H, s), 2.2~2.7 (5H, m), 2.47 (3H, s), 3.97 (3H, s), 4.13 (2H, s), 4.43 (1H, dt, J = 3 & 12Hz), 4.72 (1H, d, J = 10.5Hz), 4.9~5.9 (7H, m), 6.28 (1H, d, J = 15Hz), 8.08 (1H, d, J = 10.5Hz).

IR (KBr): 3400, 2990, 2940, 1730, 1710, 1685, 1500cm$^{-1}$.

Employing lankacidin A 8-chloroacetate as the starting material, reactions were conducted in a manner similar to that of Reference Example 29 to obtain compounds shown in Table 3.

Tabel 3 (continued)

8-position($R^3$) derivatives of Lankacidin A

| Example No. | $R^3$ | Yield (%) | m.p. (°C) | NMR: 11-Me, COCOCH$_3$, 8-H, others | IR(carbonyl region) |
|---|---|---|---|---|---|
| 12 | $Me_2(CH_2)_2SCH_2COO$- | 52 | — | 1.55(s), 2.44(s), 5.10 (m), 3.23(s, $SCH_2CO$) | 1730, 1715, 1690 |
| 13 | $Et_2N(CH_2)_2SCH_2COO$- | 28 | — | 1.57(s), 2.48(s), 5.10 (m), 3.27(s, $SCH_2CO$) | 1730, 1710, 1685 |

Example 14 Preparation of lankacidin A 8-diethylaminoacetate :

In 30 mℓ of tetrahydrofuran was dissolved 1.0 g of lankacidin A 8-iodoacetate. To the solution was added 0.5 mℓ of diethylamine, and the mixture was stirred for 1.5 hour, to which was added 200 mℓ of

ethyl acetate, followed by washing with water and saturated aqueous saline solution in sequence, then by drying over $MgSO_4$. The solvent was distilled off, and the residue was subjected to a silica gel (75 g) column chromatography. Elution was conducted with ethyl acetate - chloroform (1:1) to obtain 800 mg of the above titled compound, m.p.183 °C.

NMR (90 MHz, $CDCl_3$) $\delta$ : 1.02 (6H, t, J = 7Hz), 1.30 (3H, d, J = 6Hz), 1.35 (3H, s), 1.88 (3H, s), 2.00 (3H, s), 2.1~2.7 (5H, m), 2.43 (3H, s), 2.63 (4H, q, J = 7Hz), 3.30 (2H, s), 4.40 (1H, dt, J = 3 & 12Hz), 4.69 (1H, d, J = 10.5Hz), 5.0~5.9 (7H, m), 6.28 (1H, d, J = 15Hz), 8.07 (1H, d, J = 10.5 Hz).

IR (KBr): 3400, 2975, 2940, 1730, 1710, 1685, 1505cm$^{-1}$.

Employing lankacidin A 8-iodoacetate as the starting material, reactions were conducted in a manner similar to that of Example 14 to obtain compounds shown in Table 4.

Table 4

8-position($R^3$) derivatives of Lankacidin A

| Example No. | $R^3$ | Yield (%) | m.p. (°C) | NMR: 11-Me, $COCOCH_3$, 8-H, others | IR(carbonyl region) |
|---|---|---|---|---|---|
| 15 | $Me_2NCH_2COO$- | 82 | 191-193 (dec) | 1.53(s), 2.42(s), 5.15 (m), 3.15(br.s, $NCH_2CO$) | 1730, 1710, 1605 |
| 16 | $iPr_2NCH_2COO$- | 39 | — | 1.55(s), 2.45(s), 5.10 (m), 3.23(s, $NCH_2CO$) | 1710, 1690 |
| 17 | Mor-$CH_2COO$- | 48 | — | 1.55(s), 2.43(s), 5.12 (m), 3.18(s, $NCH_2CO$) | 1710, 1680 |

Example 18 Preparation of lankacidin A 8-[(2-dimethylaminoethyl)thio]methyl carbonate and lankacidin A 8-[S-(2-dimethylaminoethyl)]thiocarbonate :

To 22 mℓ of methanol was added 178 mg of sodium hydride (60%). To the solution was added, after an exothermic reaction and foaming finished, 316 mg of 2-dimethylaminoethane thiol. hydrochloride, then the mixture was stirred for 10 minutes. To the resultant mixture was added 1.46 g of lankacidin A 8-iodomethyl carbonate obtained in Reference Example 22 and the mixture was stirred for further one hour. To the resultant was added 500 mℓ of ethyl acetate, which was washed with aqueous saline solution (100 mℓ x 3), followed by drying on $MgSO_4$. The solvent was distilled off, and the residue was subjected to a silica gel (300 g) column chromatography. Elution was conducted with methanol - ethyl acetate (1:20) to obtain 698.5 mg of the above titled compound (methyl carbonate compound) and 83.4 mg of the above titled compound (thiocarbonate compound).

Methyl carbonate compound, m.p.99-101 °C (decomp.) :

NMR (90 MHz, $CDCl_3$) $\delta$ : 1.31 (3H, d, J = 6.5 Hz), 1.37 (3H, s), 1.55 (3H, s), 1.90 (3H, s), 2.02 (2H, s), 2.2~2.65 (7H, m), 2.26 (6H, s), 2.45 (3H, s), 2.75~2.9 (2H, m), 4.40 (1H, m), 4.72 (1H, d, J = 11Hz), 4.93 (1H, m), 5.21 (2H, s), 5.2~5.85 (6H, m), 6.29 (1H, d, J = 15Hz), 8.07 (1H, d, J = 10Hz).

IR (KBr): 3380, 2940, 1740, 1705, 1685, 1500, 1450, 1355, 1330, 1230, 1135, 1010, 960, 925cm$^{-1}$.

Thiocarbonate compound, m.p.190-191 °C (decomp.) :

NMR (90 MHz, $CDCl_3$) $\delta$ : 1.31 (3H, d, J = 6.5 Hz), 1.38 (3H, s), 1.55 (3H, s), 1.90 (3H, s), 2.03 (3H, s), 2.27 (6H, s), 2.2~2.65 (7H, m), 2.45 (3H, s), 2.9~3.05 (2H, m), 4.40 (1H, m), 4.70 (1H, d, J = 11Hz), 5.13 (1H, m), 5.2~5.85 (6H, m), 6.28 (1H, d, J = 15Hz), 8.06 (1H, d, J = 10Hz).

IR (KBr): 3390, 2935, 1725, 1705, 1685, 1500, 1450, 1355, 1235, 1135, 1010, 940cm$^{-1}$.

Employing lankacidin A 8-iodomethyl carbonate as the starting compound, reactions were conducted in a manner similar to that of Example 18 to obtain compound shown in Table 6.

Table 6 (continued)

8-position($R^3$) derivatives of Lankacidin A

| Example No. | $R^3$ | Yield (%) | m.p. (°C) | NMR: 11-Me, $COCOCH_3$, 8-H, others | IR(carbonyl region) |
|---|---|---|---|---|---|
| 19 | $AcNH(CH_2)_2SCOO-$ | — | 161-163 | 1.56(s), 2.47(s), 5.13(m) | 1730(sh), 1710, 1680 |

Example 20 Preparation of lankacidin A 8-(4-dimethylamino)butyrate :

In 30 mℓ of dichloromethane was dissolved 500 mg of lankacidin A. To the solution were added 481.3 mg of 4-(dimethylamino)butyrate•hydrochloride, 353.5 mg of triethylamine, 1.09 g of dicyclohexyl carbodiimide and 318 mg of zinc chloride, and the mixture was stirred for 1.5 hour. To the mixture were further added 160 mg of 4-(dimethylamino)butyrate•hydrochloride, 117 mg of triethylamine, 363 mg of dicyclohexylcarbodiimide and 106 mg of zinc chloride. The resultant mixture was stirred for further one hour, subjected to filtration. The solid portion was washed with 100 mℓ of chloroform. The filtrate and the washing were combined and washed with water (100 mℓ x 3), followed by drying over $MgSO_4$. The solvent was distilled off, and the residue was subjected to a silica gel (180 g) column chromatography, eluting with methanol-chloroform (1:25 then 1:8) to obtain 251.7 mg of the above titled compound as a white powdery product.

NMR (90 MHz, $CDCl_3$) δ : 1.30 (3H, d, J = 7 Hz), 1.37 (3H, s), 1.54 (3H, s), 1.90 (3H, s), 1.7~1.95 (2H, m), 2.02 (3H, s), 2.24 (6H, s), 2.2~2.55 (9H, m), 2.44 (3H, s), 4.40 (1H, m), 4.70 (1H, d, J = 11Hz), 5.07 (1H, m), 5.25~5.9 (6H, m), 6.29 (1H, d, J = 15Hz), 8.07 (1H, d, J = 10Hz).

IR (KBr): 3400, 2940, 1725, 1710, 1690, 1500, 1360, 1230, 1160, 1135, 1015, 960cm$^{-1}$.

A reaction similar to that in Example 20 gave the compound of Example 21 as shown in Table 7.

Table 7

8-position($R^3$) derivatives of Lankacidin A

| Example No. | $R^3$ | Yield (%) | m.p. (°C) | NMR: 11-Me, $COCOCH_3$, 8-H, others | IR(carbonyl region) |
|---|---|---|---|---|---|
| 21 | $Me_2N(CH_2)_2COO$- | 55.9 | 177-179 (dec) | 1.55(s), 2.44(s), 5.08 (m), 2.23(s, $NMe_2$) | 1730, 1715, 1690, |

Reference Example 30 Preparation of lankacidin C 8-N-methyl carbamate :

In a mixture of 24.3 mℓ of tetrahydrofuran and 24.3 mℓ of methanol was dissolved 135.5 mg of lankacidin A 8-N-methyl carbamate, to which was added a solution of 2.2 g of the esterase prepared in Reference Example 11 in 48.6 mℓ of water. The whole mixture was stirred for one hour, and then subjected to extraction with 47.2 mℓ of chloroform. The organic layer was washed with an aqueous NaCl solution and dried over $MgSO_4$. The solvent was distilled off. The residue was subjected to a silica gel (25 g) column chromatography, eluting with ethyl acetate. The eluate was fractionated by 5 g each. The 12th~17th fractions were combined and subjected to distillation to obtain 77.4 mg of the above titled compound.

NMR (90 MHz, $CDCl_3$) δ : 1.26 (3H, d, J = 7Hz), 1.37 (3H, s), 1.54 (3H, s), 1.89 (3H, s), 2.2~2.6 (5H, m), 2.44 (3H, s), 2.77 (3H, d, J = 5Hz), ~ 4.3 (1H, m), 4.42 (1H, m), 4.69 (1H, d, J = 11Hz), ~ 4.7 (1H, m), 5.2~5.95 (5H, m), 6.17 (3H, d, J = 15Hz), 8.06 (1H, d, J = 10Hz).

IR (KBr): 1720 (sh.), 1704, 1682 (sh.), 1500, 1254, 1130, 960cm$^{-1}$.

Employing corresponding lankacidin A 8-derivatives as starting compounds, reactions similar to that in Reference Example 30 gave the compounds shown in Table 8.

Table 8 (continued)

8-position($R^3$) derivatives of Lankacidin C

| Example No. | $R^3$ | Yield (%) | m.p. (°C) | NMR: 11-Me, $COCOCH_3$, 8-H, others | IR(carbonyl region) |
|---|---|---|---|---|---|
| 22 | TeM-$SCH_2OCOO$- | 97 | 136-138 (dec) | 1.54(s), 2.44(s), 4.93 (m), 3.93(s, NMe) | 1745, 1705, 1685 |
| 23 | $Me_2N(CH_2)_2OCOO$- | 74.6 | 151-154 (dec) | 1.55(s), 2.44(s), 4.92 (m), 2.23(s, $NMe_2$) | 1745, 1705, 1680 |
| 24 | $Me_2N(CH_2)_2SCOO$- | 52.6 | — | 1.54(s), 2.44(s), 5.11 (m), 2.25(s, $NMe_2$) | 1750, 1700, 1690 (sh) |
| 25 | ThMe-$SCH_2OCOO$- | 88.8 | — | 1.56(s), 2.47(s), 4.95 (m), 3.48(s, OMe) | 1750, 1710, 1685 |
| 26 | TeM-$SCH_2COO$- | 74 | — | 1.55(s), 2.45(s), 5.09 (m), 3.98(s, NMe) | 1740, 1705, 1680 |

Table 8 (continued)

8-position($R^3$) derivatives of Lankacidin C

| Example No. | $R^3$ | Yield (%) | m.p. (°C) | NMR: 11-Me, $COCOCH_3$, 8-H, others | IR(carbonyl region) |
|---|---|---|---|---|---|
| 27 | $EtNHCOO-$ | 65 | 201-203 (dec) | 1.54(s), 2.46(s), 4.97 (m), 1.12(t, J=7Hz, $CH_3CH_2$) | 1740(sh), 1704, 1680(sh) |
| 28 | $MeOCOO-$ | 75 | 201-202 (dec) | 1.55(s), 2.45(s), 4.92(m), 3.77(s, MeO) | 1742, 1706, 1678, |
| 29 | $Me_2N(CH_2)_2SCH_2COO-$ | 60 | – | 1.54(s), 2.43(s), 5.10 (m), 3.22(s, $SCH_2CO$) | 1710, 1685 |
| 30 | $Et_2N(CH_2)_2SCH_2COO-$ | 50 | – | 1.55(s), 2.45(s), 5.09 (m), 3.25(s, $SCH_2CO$) | 1725(sh), 1705, 1690 |
| 31 | Mor-COO- | 67 | – | 1. 56(s), 2.47(s), 5.01 (m), ~3.55(m, Mor-$CH_2$) | 1740(sh), 1700, 1685(sh) |

Table 8 (continued)

8-position($R^3$) derivatives of Lankacidin C

| Example No. | $R^3$ | Yield (%) | m.p. (°C) | NMR: 11-Me, $COCOCH_3$, 8-H, others | IR(carbonyl region) |
|---|---|---|---|---|---|
| 32 | H-Pipe-COO- | 64 | – | 1.53(s), 2.43(s), 4.97 (m), 2.80(br.s, $C\underline{H}_2NHCH_2$) | 1740, 1700, 1688(sh) |
| 33 | 2-Py-Pipe-COO- | 65 | – | 1.55(s), 2.42(s), 5.01 (m), 3.55(s, Pipe-$CH_2$) | 1740, 1700, 1682 (sh) |
| 34 | $Me_2NCH_2COO$- | 75 | 204-208 (dec) | 1.53(s), 2.43(s), 5.15 (m), 2.23(s, $NMe_2$) | 1740, 1720(sh), 1705, 1675 |
| 35 | $Et_2NCH_2COO$- | 73 | 193-196 (dec) | 1.55(s), 2.45(s), 5.14 (m), 3.30(s, $NCH_2CO$) | 1740(sh), 1705, 1685 |
| 36 | 3-Py-$CH_2NHCOO$- | 70 | 193-195 | 1.49(s), 2.38(s), 5.12 (m), 4.23(d, J = 6Hz, $NCH_2$) | 1740(sh), 1708 1682(sh) |

Table 8 (continued)

8-position($R^3$) derivatives of Lankacidin C

| Example No. | $R^3$ | Yield (%) | m.p. (°C) | NMR: 11-Me, $COCOCH_3$, 8-H, others | IR(carbonyl region) |
|---|---|---|---|---|---|
| 37 | Me-Pipe-COO- | 79 | 201-203 (dec) | 1.54(s), 2.44(s), 4.99 (m), 2.27(s, NMe) | 1744, 1700, 1688 (sh) |
| 38 | $AcNH(CH_2)_2SCOO-$ | 75.9 | 175-178 | 1.54(s), 2.44(s), 5.11 (m), 1.94(s, AcN) | 1750, 1710, 1690 |
| 39 | $AcNH(CH_2)_2SCH_2OCOO-$ | 19.5 | 108-110 | 1.55(s), 2.44(s), 4.92 (m), 1.97(s, AcN) | 1740, 1710, 1670 |
| 40 | $Me_2N-(CH_2)_2COO-$ | 41.8 | 184-186 (dec) | 1.54(s), 2.44(s), 5.08 (m), 2.23(s, $NMe_2$) | 1720, 1710, 1685 |
| 41 | $Me_2N-(CH_2)_3COO-$ | 71.3 | 163-165 | 1.55(s), 2.44(s), 5.07 (m), 2.24(s, $NMe_2$) | 1725, 1710, 1690 |
| 42 | $2-Py-Pipe-CH_2COO-$ | 64 | — | 1.57(s), 2.47(s), 5.12 (m), 3.27(br.s, $-CH_2COO$) | 1740(sh), 1705, 1680 |

Table 8 (continued)

8-position($R^3$) derivatives of Lankacidin C

| Example No. | $R^3$ | Yield (%) | m.p. (°C) | NMR: 11-Me, $COCOCH_3$, 8-H, others | IR(carbonyl region) |
|---|---|---|---|---|---|
| 43 | $Cl(CH_2)_2NHCH_2COO-$ | 59 | – | 1.56(s), 2.46(s), 5.12(m), 3.45(br.s, $NCH_2CO$) | 1730, 1700, 1680 |
| 44 | $F(CH_2)_2NHCH_2COO-$ | 72 | – | 1.56(s), 2.45(s), 5.12(m), 3.46(br.s, $NCH_2CO$) | 1730, 1700, 1680 |
| 45 | $2-Py-(CH_2)_2NHCOO-$ | 65 | – | 1.50(s), 2.40(s), 4.90(m), 2.93(t, J=7Hz, $PyC\underline{H}_2CH_2$) (DM) | 1746(sh), 1708, 1682(sh) |
| 46 | $2-Py-CH_2NHCOO-$ | 70 | 192-194 (dec) | 1.54(s), 2.45(s), 5.01(m), 4.47(d, J=6Hz, $NHC\underline{H}_2$) | 1740(sh), 1704, 1684(sh) |
| 47 | $4-Py-CH_2NHCOO-$ | 76 | 215-217 (dec) | 1.51(s), 2.42(s), 4.95(m), 4.27(d, J=6Hz, $NC\underline{H}_2$) | 1740(sh), 1718(sh), 1704, 1674 |

Table 8 (continued)

8-position($R^3$) derivatives of Lankacidin C

| Example No. | $R^3$ | Yield (%) | m.p. (°C) | NMR: 11-Me, $COCOCH_3$, 8-H, others | IR(carbonyl region) |
|---|---|---|---|---|---|
| 48 | $PhCH_2Pipe$-COO- | 62 | – | 1.54(s), 2.44(s), 4.97 (m), 3.50(s, $PhCH_2N$) | 1740, 1700, 1685 |
| 49 | 4-Py-Pipe-COO- | 54.7 | 180-182 (dec) | 1.55(s), 2.44(s), 5.01 (m), 3.25-3.7(m, Pipe-$CH_2$) | 1710, 1685 |
| 50 | $PrNHCH_2COO$- | 51 | – | 1.57(s), 2.47(s), 5.09 (m), 0.90(t, J = 7Hz, $CH_3(CH_2)_2$) | 1705, 1685 |
| 51 | $N_3CH_2COO$- | 59 | – | 1.57(s), 2.47(s), 5.17 (m), 3.87(s, $N_3CH_2$) | 1730, 1705, 1675 |
| 52 | $iPr_2NCH_2COO$- | 80 | – | 1.55(s), 2.45(s), 5.10 (m), 1.00(s, $Me_2CH$ × 2) | 1740, 1705, 1685 |

Table 8 (continued)

8-position($R^3$) derivatives of Lankacidin C

| Example No. | $R^3$ | Yield (%) | m.p. (°C) | NMR: 11-Me, $COCOCH_3$, 8-H, others | IR(carbonyl region) |
|---|---|---|---|---|---|
| 53 | $iPrNHCH_2COO-$ | 65 | – | 1.55(s), 2.44(s), 5.09 (m), 3.38(br.s, $NCH_2CO$) | 1730, 1700 |
| 54 | $Mor-CH_2COO-$ | 71 | – | 1.55(s), 2.45(s), 5.10 (m), 3.20(s, $NCH_2CO$) | 1735, 1700, 1660 |
| 55 | $4-Py-Pipe-CH_2COO-$ | 50 | – | 1.53(s), 2.44(s), 5.10 (m), 3.27(br.s, $CH_2CO$) | 1740, 1705, 1600 |

R': *1 = $3-CH_3C(=NOCH_3)CONH$

Example 56 Preparation of O(8)-(4-methylpiperazino) carbonyl lankacidin A hydrochloride :

In 6.4 mℓ of tetrahydrofuran was dissolved 200 mg of O(8)-(4-methylpiperazino)carbonyl lankacidin A. To the solution was added 0.139 mℓ of 1N HCℓ, which was left standing for 10 minutes. The solvent was

distilled off. The residual glassy substance was treated with ether, and pulverized. The powdery product was collected by filtration and dried to obtain 203.7 mg of the above titled compound.

NMR (90 MHz, $CDCl_3$) δ : 1.31 (3H, d, J = 7Hz), 1.38 (3H, s), 1.55 (3H, s), 1.90 (3H, s), 2.02 (3H, s), 2.1~2.65 (5H, m), 2.44 (3H, s), 2.83 (3H, s), 3.15 (4H, br.), 4.0 (4H, br.), 4.43 (1H, m), 4.72 (1H, d, J = 11Hz), 5.00 (1H, m),5.2~5.9 (6H, m), 6.28 (1H, d, J = 15Hz), 8.07 (1H, d, J = 10Hz).

IR (KBr): 1740 (sh.), 1720 (sh.), 1700, 1460 (sh.), 1420, 1254, 962$cm^{-1}$.

Employing as starting materials free amino compounds of corresponding lankacidin A or C derivatives, reactions similar to that in Example 56 were conducted to obtain the respective hydrochloride shown in Table 9.

Table 9

Hydrochloride of Lankacidin Derivative

| Example No. | $R^3$ | $R^4$ | Yield (%) | m.p. (°C) | NMR: 11-Me, $COOCH_3$, 8-H, ($CDCl_3$) others | IR (KBr) (carbonyl region) |
|---|---|---|---|---|---|---|
| 57 | Me-Pipe-COO- | OH | 99 | – | 1.54(s), 2.43(s), 4.98 (m), 2.83(s, NMe) (DM) | 1734(sh), 1696, 1680(sh) |
| 58 | $Me_2NCH_2COO$- | OAc | 107 | 156-163 (dec) | 1.57(s), 2.45(s), 5.10 (m), 3.00(br.s, $NMe_2$) | 1730, 1705 |
| 59 | $Me_2NCH_2COO$- | OH | 94 | 182-195 (dec) | 1.53(s), 2.43(s), 5.09 (m), 3.00(br.s, $NMe_2$) | 1740, 1705 |
| 60 | $Et_2NCH_2COO$- | OAc | 94 | 154-160 (dec) | 1.57(s), 2.47(s), 5.11 (m), 3.92(br.s, $NCH_2CO$) | 1740, 1705 |
| 61 | $Et_2NCH_2COO$- | OH | 98 | 159-171 (dec) | 1.57(s), 2.45(s), 5.08 (m), 3.90(br.s, $NCH_2CO$) | 1740, 1700 |

Table 9 (continued)

Hydrochloride of Lankacidin Derivative

| Example No. | $R^3$ | $R^4$ | Yield (%) | m.p. (°C) | NMR: 11-Me, $COCOCH_3$, 8-H, ($CDCl_3$) others | IR (KBr) (carbonyl region) |
|---|---|---|---|---|---|---|
| 62 | 2-Py-Pipe-COO- | OAc | 99 | — | 1.57(s), 2.46(s), 5.03 (m), 3.77 & 3.90(each br.s, Pipe-$CH_2$) | 1720(sh), 1700, 1682(sh), 1632, 1602 |
| 63 | 2-Py-Pipe-COO- | OH | 94 | 214 (dec) | 1.55(s), 2.44(s), 5.00 (m), 3.77 & 3.90(each br.s, Pipe-$CH_2$) | 1730, 1692, 1680(sh), 1632, 1604 |
| 64 | $Me_2N(CH_2)_3COO$- | OAc | 96 | 158-161 (dec) | 1.55(s), 2.45(s), 5.05 (m), 2.80(s, $NMe_2$) | 1730, 1710, 1690(sh) |
| 65 | $Me_2N(CH_2)_3COO$- | OH | 98 | 157-160 (dec) | 1.55(s), 2.45(s), 5.04 (m), 2.81(s, $NMe_2$) | 1730, 1710, 1690(sh) |
| 66 | $Me_2N(CH_2)_2SCH_2COO$- | OAc | 77 | — | 1.54(s), 2.45(s), 5.06 (m), 2.73(s, $NMe_2$) | 1740, 1705 |

Table 9 (continued)

Hydrochloride of Lankacidin Derivative

| Example No. | $R^3$ | $R^4$ | Yield (%) | m.p. (°C) | NMR: 11-Me, $COCOCH_3$, 8-H, ($CDCl_3$) others | IR (KBr) (carbonyl region) |
|---|---|---|---|---|---|---|
| 67 | $Me_2N(CH_2)_2SCH_2COO$- | OH | 98 | — | 1.57(s), 2.47(s), 5.04 (m), 2.83(br.s, $NMe_2$) | 1740, 1705 |
| 68 | 4-Py-Pipe-COO- | OAc | 96 | 181-183 (dec) | 1.56(s), 2.45(s), 5.03 (m), 3.74(br.s, Pipe-$CH_2$) | 1720(sh), 1700, 1690(sh), 1640 |
| 69 | 4-Py-Pipe-COO- | OH | 91 | > 300 | 1.55(s), 2.44(s), 5.00 (m), 3.72(br.s, Pipe-$CH_2$) | 1700, 1690(sh), 1640 |
| 70 | $iPr_2NCH_2COO$- | OAc | 95 | — | 1.60(s), 2.47(s), 5.09 (m), 3.90(br.s, $NCH_2CO$) | 1725, 1705, 1680 |
| 71 | $iPr_2NCH_2COO$- | OH | 98 | — | 1.58(s), 2.47(s), 5.08 (m), 3.88(br.s, $NCH_2CO$) | 1725(sh), 1705, 1680(sh) |

## Claims

### Claims for the following Contracting States : BE, CH, DE, FR, GB, GR, IT, LU, LI, NL, SE

**1.** A compound of the general formula:

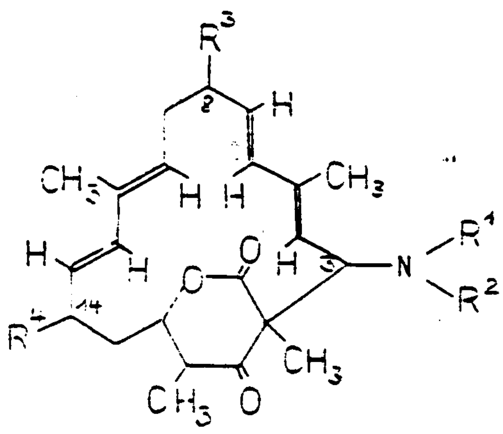

wherein
(i)
$R^1$ stands for a hydrogen atom,
$R^2$ stands for a group of the formula:

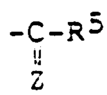

wherein Z stands for oxygen or sulfur, and $R^5$ stands for an $C_1$-$C_5$-alkanoyl group, an α-hydroxy $C_{1-4}$alkyl group, or
(ii)
$R^1$ and $R^2$, taken together, stand for a group of the formula

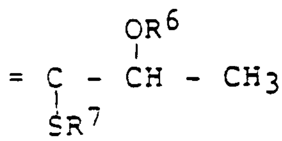

wherein $R^6$ stands for a $C_{1-5}$ alkanoyl group and $R^7$ stands for a $C_{1-4}$alkyl group;
$R^3$ is
(1) -OCOO-$C_{1-4}$alkyl,
(2) -OCOS-$C_{1-4}$alkyl substituted with ω-$C_{2-4}$alkanoylamino,
(3) -$OCOR^{14}$ wherein $R^{14}$ is a $C_{1-4}$alkyl group substituted at the terminus with (a) di($C_{1-4}$alkyl)amino or (b) a 5 to 8 membered heterocyclic group containing 1 to 4 hetero atoms of oxygen, sulfur or nitrogen, and having optionally an intermediate sulfur atom;
(4) -$OCONR^{15}R^{16}$ wherein $R^{15}$ and $R^{16}$ respectively are a $C_{1-5}$alkyl group or together form a heterocyclic group containing nitrogen atoms which may be substituted by (1) $C_{1-4}$alkyl or (2) pyridyl; $R^4$ is hydroxy or a $C_{1-4}$alkanoyloxy group;
or a salt thereof.

**2.** The compound according to claim 1, wherein Z is sulfur and $R^5$ is acetyl.

**3.** The compound according to claim 1, wherein Z is oxygen and $R^5$ is acetyl.

4. The compound according to claim 1, wherein the salt is an acid addition salt.

5. The compound according to claim 1, wherein $R^3$ is $-OCONR^{15}R^{16}$ wherein $R^{15}$ and $R^{16}$ respectively are a $C_{1-5}$alkyl group or form a heterocyclic group containing nitrogen atoms which may be substituted by (1) $C_{1-4}$alkyl or (2) pyridyl.

6. The compound according to claim 1, wherein $R^4$ is hydroxy.

7. A compound selected from 3-(2-oxo-1-thioxopropylamino)lankone 8-acetate, O(8)-(4-methylpiperazino)-carbonyl-lankacidin A, O(8)-[4-(2-pyridyl)piperazino] carbonyl-lankacidin A,
lancacidin C 8-dimethylaminoacetate,
lankaoidin C 8-diethylaminoacetate,
lankacidin A 8-[(2-dimethylaminoethyl)thio]methylcarbonate,
lankacidin C 8-[(2-dimethylaminoethyl)thio]methylcarbonate,
O(8)-(4-methylpiperazino)carbonyl-lankacidin C,
lankacidin A 8-[S-(2-dimethylaminoethyl)thiocarbonate,
lankacidin C 8-[S-(2-dimethylaminoethyl)thiocarbonate,
lankacidin A 8-diethylaminoacetate,
lankacidin A 8-diisopropylaminoacetate,
lankacidin C 8-diisopropylaminoacetate,
lankacidin C 8-(3-dimethylamino)propionate,
lankacidin C 8-(3-dimethylamino)butyrate,
lankacidin C 8-morpholinoacetate,
O(8)-[4-(4-pyridyl)piperazino)carbonyl-lankacidin C,
O(8)-[4-(2-pyridyl)piperazino)carbonyl-lankacidin C,
or a salt thereof.

8. A compound, selected from O(8)-(4-benzylpiperazino)carbonyllankacidin C,
3-(2-acetoxyacrylamido)lankone 8, 14-diacetate
O(8)-(2-acetamidoethylthio)methoxycarbonyl lankacidin C,
lankacidin C 8-(2-chloroethyl)aminoacetate,
lankacidin C 8-(2-fluoroethyl)aminoacetate,
O(8)-[2-(2-pyridyl)ethylamino]carbonyl lankacidin C,
O(8)-[(2-pyridyl)methylamino]carbonyl lankacidin C,
lankacidin C 8-propylaminoacetate,
lankacidin C 8-isopropylaminoacetate,
lankacidin C 8-(4-(4-pyridyl)piperazino)acetate,
O(8)-[(5-methoxymethyl-1,3,4-thiadiazol-2-yl)thio] methoxycarbonyl-lankacidin C,
O(8)-(4-pyridylmethylamino)carbonyl-lankacidin C,
lankacidin C 8-azidoacetate,
8-dehydroxy-8-(dimethyl amino-1H-tetrazol-5-yl)thio-lankacidin C
O(8)-[(1-methyl-1H-tetrazol-5-yl)thio]methoxy carbonyl-lankacidin C,
lankacidin C 8-[(1-methyl-1H-tetrazol-5-yl)thio]-acetate,
lankacidin C 8-[4-(2-pyridyl)piperazino]acetate,
O(8)-ethylaminocarbonyl lankacidin C,
O(8)-morpholinocarbonyl lankacidin C,
O(8)-(3-pyridyl)methylaminocarbonyl-lankacidin C,
or a salt thereof.

9. A method for producing the compound as claimed in claims 1 - 8 which comprises subjecting a compound of the formula:

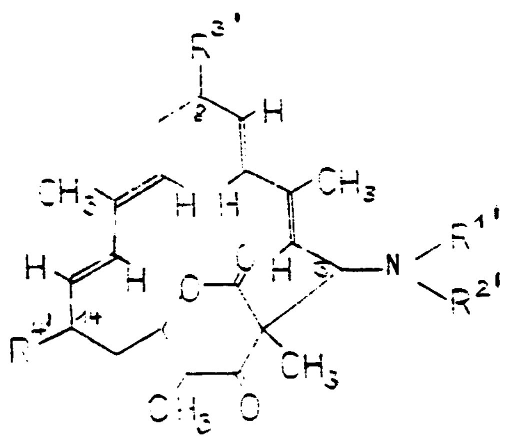

wherein $R^{1'}$, $R^{2'}$, $R^{3'}$ and $R^{4'}$ are respectively the same as $R^1$, $R^2$, $R^3$ and $R^4$ defined above or the groups convertible thereto, to alkylation, acylation, hydrolysis, thioamidation, halogenation, azidation, reaction with amines, reaction with a thiol or a salt, and/or reaction with isocyanate.

**Claims for the following Contracting States : AT, ES**

**1.** A Process for producing a compound of the general formula

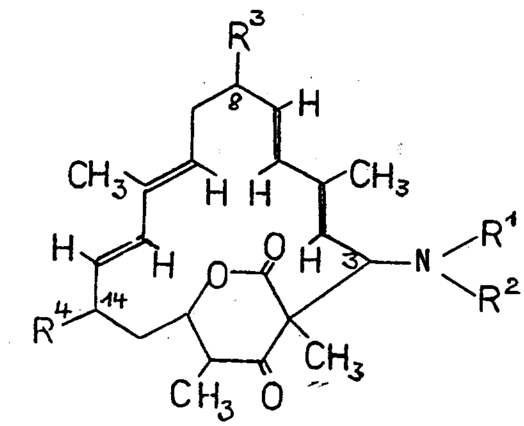

wherein
(i)
$R^1$ stands for a hydrogen atom,
$R^2$ stands for a group of the formula:

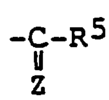

wherein Z stands for oxygen or sulfur, and $R^5$ stands for an $C_1$-$C_5$-alkanoyl group, an $\alpha$-hydroxy$C_{1-4}$alkyl group, or
(ii)
$R^1$ and $R^2$, taken together, stand for a group of the formula

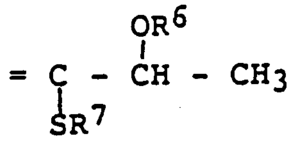

wherein $R^6$ stands for a $C_{1-5}$ alkanoyl group and $R^7$ stands for a $C_{1-4}$alkyl group;
$R^3$ is

(1) -OCOO-$C_{1-4}$alkyl,
(2) -OCOS-$C_{1-4}$alkyl substituted with $\omega$-$C_{2-4}$alkanoylamino,
(3) -$OCOR^{14}$ wherein $R^{14}$ is a $C_{1-4}$ alkyl group substituted at the terminus with (a) di($C_{1-4}$ alkyl)-amino or (b) a 5 to 8 membered heterocyclic group containing 1 to 4 hetero atoms of oxygen, sulfur or nitrogen, and having optionally an intermediate sulfur atom;
(4) -$OCONR^{15}R^{16}$ wherein $R^{15}$ and $R^{16}$ respectively are a $C_{1-5}$alkyl group or form a heterocyclic group containing nitrogen atoms which may be substituted by (1) $C_{1-4}$alkyl or (2) pyridyl; $R^4$ is hydroxy or a $C_{1-4}$alkanoyloxy group;

or a salt thereof,
which comprises subjecting a compound of the formula:

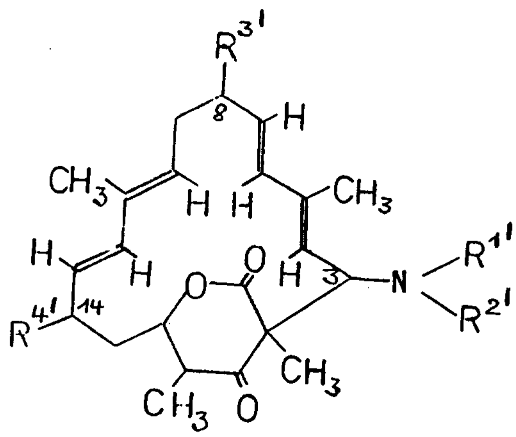

wherein $R^{1'}$, $R^{2'}$, $R^{3'}$ and $R^{4'}$ are respectively the same as $R^1$, $R^2$, $R^3$ and $R^4$ defined above or the groups convertible thereto, to alkylation, acylation, hydrolysis, thioamidation, halogenation, azidation, reaction with amines, reaction with a thiol or a salt, and/or reaction with isocyanate.

**2.** The process according to claim 1, wherein Z is sulfur and $R^5$ is acetyl.

**3.** The process according to claim 1, wherein Z is oxygen and $R^5$ is acetyl.

**4.** The process according to claim 1, wherein the salt is an acid addition salt.

**5.** The process according to claim 1, wherein $R^3$ is -$OCONR^{15}R^{16}$ wherein $R^{15}$ and $R^{16}$ respectively are a $C_{1-5}$alkyl group or form a heterocyclic group containing nitrogen atoms which may be substituted by (1) $C_{1-4}$alkyl or (2) pyridyl.

**6.** The process according to claim 1, wherein $R^4$ is hydroxy.

**7.** Process according to claim 1 for preparing a compound selected from 3-(2-oxo-1-thioxopropylamino)-lankone 8-acetate,
O(8)-(4-methylpiperazino)carbonyl-lankacidin A,
O(8)-[4-(2-pyridyl)piperazino] carbonyl-lankacidin A,
lancacidin C 8-dimethylaminoacetate,
lankacidin C 8-diethylaminoacetate,

lankacidin A 8-[(2-dimethylaminoethyl)thio]methylcarbonate,
lankacidin C 8-[(2-dimethylaminoethyl)thio]methylcarbonate,
O(8)-(4-methylpiperazino)carbonyl-lankacidin C,
lankacidin A 8-[S-(2-dimethylaminoethyl)thiocarbonate,
lankacidin C 8-[S-(2-dimethylaminoethyl)thiocarbonate,
lankacidin A 8-diethylaminoacetate,
lankacidin A 8-diisopropylaminoacetate,
lankacidin C 8-diisopropylaminoacetate,
lankacidin C 8-(3-dimethylamino)propionate,
lankacidin C 8-(3-dimethylamino)butyrate,
lankacidin C 8-morpholinoacetate,
O(8)-[4-(4-pyridyl)piperazino)carbonyl-lankacidin C,
O(8)-[4-(2-pyridyl)piperazino)carbonyl-lankacidin C,
or a salt thereof.

**8.** Process according to claim 1 for preparing a compound selected from O(8)-(4-benzylpiperazino)-carbonyllankacidin C,
3-(2-acetoxyacrylamido)lankone 8, 14-diacetate
O(S)-(2-acetamidoethylthio)methoxycarbonyl lankacidin C,
lankacidin C 8-(2-chloroethyl)aminoacetate,
lankacidin C 8-(2-fluoroethyl)aminoacetate,
O(8)-[2-(2-pyridyl)ethylamino]carbonyl lankacidin C,
O(8)-[(2-pyridyl)methylamino]carbonyl lankacidin C,
lankacidin C 8-propylaminoacetate,
lankacidin C 8-isopropylaminoacetate,
lankacidin C 8-(4-(4-pyridyl)piperazino)acetate,
O(8)-[(5-methoxymethyl-1,3,4-thiadiazol-2-yl)thio] methoxycarbonyl-lankacidin C,
O(8)-(4-pyridylmethylamino)carbonyl-lankacidin C,
lankacidin C 8-azidoacetate,
8-dehydroxy-8-(dimethyl amino-1H-tetrazol-5-yl)thio-lankacidin C
O(8)-[(1-methyl-1H-tetrazol-5-yl)thio]methoxy carbonyl-lankacidin C,
lankacidin C 8-[(1-methyl-1H-tetrazol-5-yl)thio]-acetate,
lankacidin C 8-[4-(2-pyridyl)piperazino]acetate,
O(8)-ethylaminocarbonyl lankacidin C,
O(8)-morpholinocarbonyl lankacidin C,
O(8)-(3-pyridyl)methylaminocarbonyl-lankacidin C,
or a salt thereof.

## Patentansprüche

## Patentansprüche für folgende Vertragsstaaten : BE, CH, DE, FR, GB, GR, IT, LU, LI, NL, SE

**1.** Verbindung der allgemeinen Formel:

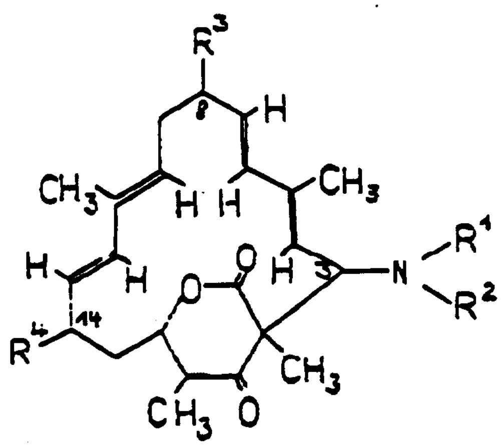

worin
(i)
$R^1$ für ein Wasserstoffatom steht,
$R^2$ für eine Gruppe der Formel

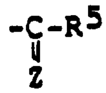

steht, worin Z für Sauerstoff oder Schwefel steht und $R^5$ für eine $C_1$-$C_5$-Alkanoylgruppe, eine $\alpha$-Hydroxy-$C_{1-4}$-Alkylgruppe steht, oder
(ii)
$R^1$ und $R^2$ gemeinsam für eine Gruppe der Formel

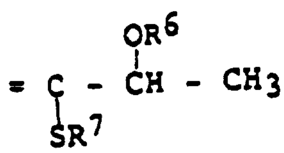

stehen, worin $R^6$ für eine $C_{1-5}$-Alkanoylgruppe steht und $R^7$ für eine $C_{1-4}$-Alkylgruppe steht;
$R^3$
(1) -OCOO-$C_{1-4}$-Alkyl,
(2) -OCOS-$C_{1-4}$-Alkyl, substituiert mit $\omega$-$C_{2-4}$-alkanoylamino,
(3) -$OCOR^{14}$, worin $R^{14}$ eine $C_{1-4}$-Alkylgruppe ist, die am Terminus durch (a) di($C_{1-4}$-Alkyl)amino oder (b) eine 5- bis 8-gliedrige heterozyklische Gruppe substituiert ist, die 1 bis 4 Heteroatome ausgewählt aus Sauerstoff, Schwefel oder Stickstoff enthält, und wahlweise ein dazwischenliegendes Schwefelatom aufweist,
(4) -$OCONR^{15}R^{16}$ ist, worin $R^{15}$ und $R^{16}$ jeweils eine $C_{1-5}$-Alkylgruppe sind oder gemeinsam eine heterozyklische Gruppe bilden, die Stickstoffatome enthält, die durch (1) $C_{1-4}$-Alkyl oder (2) Pyridyl substituiert sein können;
$R^4$ Hydroxy oder eine $C_{1-4}$-Alkanoyloxygruppe ist;
oder ein Salz davon.

**2.** Verbindung nach Anspruch 1, worin Z Schwefel ist und $R^5$ Acetyl ist.

**3.** Verbindung nach Anspruch 1, worin Z Sauerstoff ist und $R^5$ Acetyl ist.

**4.** Verbindung nach Anspruch 1, worin das Salz ein Säureadditionssalz ist.

**5.** Verbindung nach Anspruch 1, worin $R^3$ -$OCONR^{15}R^{16}$ ist, worin $R^{15}$ und $R^{16}$ jeweils eine $C_{1-5}$-Alkylgruppe sind oder eine heterozyklische Gruppe bilden, die Stickstoffatome enthält, die durch (1) $C_{1-4}$-Alkyl oder (2) Pyridyl substituiert sein können.

**6.** Verbindung nach Anspruch 1, worin $R^4$ Hydroxy ist.

**7.** Verbindung, ausgewählt aus
3-(2-Oxo-1-thioxopropylamino)lankon 8-acetat,
O(8)-(4-Methylpiperazino)carbonyl-lankacidin A,
O(8)-[4-(2-Pyridyl)piperazino] carbonyl-lankacidin A,
Lankacidin C 8-dimethylaminoacetat,
Lankacidin C 8-diäthylaminoacetat,
Lankacidin A 8-[(2-dimethylaminoäthyl)thio]methylcarbonat,
Lankacidin C 8-[(2-dimethylaminoäthyl)thio]methylcarbonat,
O(8)-(4-methylpiperazino)carbonyl-lankacidin C,
Lankacidin A 8-[S-(2-dimethylaminoäthyl)thiocarbonat,
Lankacidin C 8-[S-(2-dimethylaminoäthyl)thiocarbonat,

Lankacidin A 8-diäthylaminoacetat,
Lankacidin A 8-diisopropylaminoacetat,
Lankacidin C 8-diiosypropylaminoacetat,
Lankacidin C 8-(3-dimethylamino)propionat,
Lankacidin C 8-(3-dimethylamino)butyrat,
Lankacidin C 8-morpholinoacetat,
O(8)-[4-(4-Pyridyl)piperazino)carbonyl-lankacidin C,
O(8)-[4-(2-Pyridyl)piperazino)carbonyl-lankacidin C,
oder ein Salz davon.

**8.** Verbindung, ausgewählt aus
O(8)-(4-Benzylpiperazino)carbonyl-lankacidin C,
3-(2-Acetoxyacrylamido)lankon-8,14-diacetat
O(8)-(2-Acetamidoäthylthio)methoxycarbonyl-lankacidin C,
Lankacidin C 8-(2-chloräthyl)aminoacetat,
Lankacidin C 8-(2-fluoräthyl)aminoacetat,
O(8)-[2-(2-Pyridyl)äthylamino]carbonyl-lankacidin C,
O(8)-[(2-Pyridyl)methylamino]carbonyl-lankacidin C,
Lankacidin C 8-propylaminoacetat,
Lankacidin C 8-isopropylaminoacetat,
Lankacidin C 8-(4-(4-pyridyl)piperazino)acetat,
O(8)-[(5-Methoxymethyl-1,3,4,-thiadiazol-2-yl)thio]methoxycarbonyllankacidin C,
O(8)-(4-Pyridylmethylamino)carbonyl-lankacidin C,
Lankacidin C 8-azidoacetat,
8-Dehydroxy-8-(dimethylamino-1H-tetrazol-5-yl)thio-lankacidin C,
O(8)-[(1-Methyl-1H-tetrazol-5-yl)thio]methoxycarbonyl-lankacidin C,
Lankacidin C 8-[(1-methyl-1H-tetrazol-5-yl)thio]-acetat,
Lankacidin C 8-[4-(2-pyridyl)piperazino]acetat,
O(8)-Äthylaminocarbonyl-lankacidin C,
O(8)-Morpholinocarbonyl-lankacidin C,
O(8)-(3-Pyridyl)methylaminocarbonyl-lankacidin C,
oder ein Salz davon.

**9.** Verfahren zur Herstellung der Verbindung nach einem der Ansprüche 1 bis 8, die das Unterwerfen einer Verbindung der Formel:

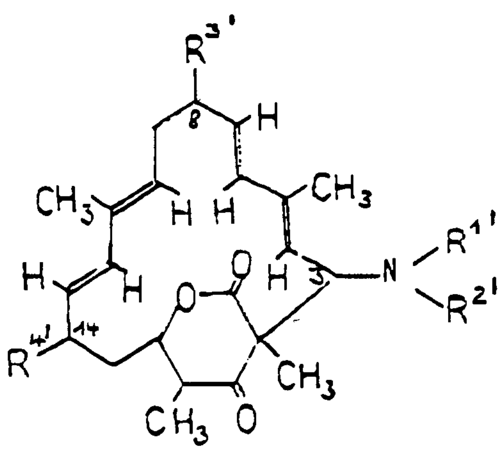

worin $R^{1'}$, $R^{2'}$, $R^{3'}$ und $R^{4'}$ jeweils die gleiche Bedeutung haben wie oben für $R^1$, $R^2$, $R^3$ und $R^4$ angegeben oder gleich den in sie umwandelbaren Gruppen sind, der Alkylierung, Acylierung, Hydrolyse, Thioamidierung, Halogenierung, Azidierung, Umsetzung mit Aminen, Umsetzung mit einem Thiol oder einem Salz und/oder Umsetzung mit Isocyanat umfaßt.

**Patentansprüche für folgende Vertragsstaaten : AT, ES**

**1.** Verfahren zur Herstellung einer Verbindung der allgemeinen Formel:

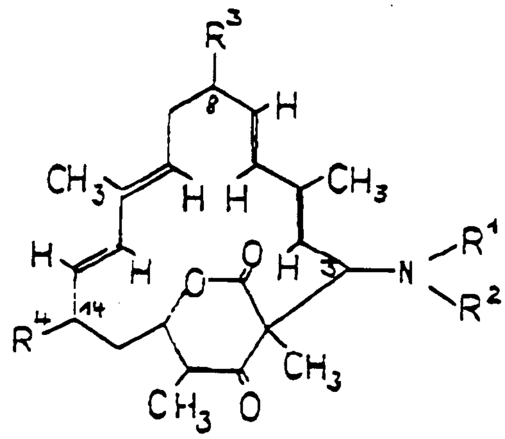

worin
(i)
$R^1$ für ein Wasserstoffatom steht,
$R^2$ für eine Gruppe der Formel

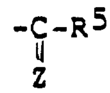

steht, worin Z für Sauerstoff oder Schwefel steht und $R^5$ für eine $C_1$-$C_5$-Alkanoylgruppe, eine $\alpha$-Hydroxy-$C_{1-4}$-Alkylgruppe steht, oder
(ii)
$R^1$ und $R^2$ gemeinsam für eine Gruppe der Formel

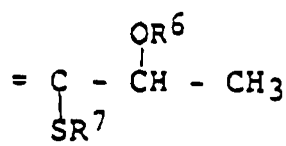

stehen, worin $R^6$ für eine $C_{1-5}$-Alkanoylgruppe steht und $R^7$ für eine $C_{1-4}$-Alkylgruppe steht;
$R^3$

(1) -OCOO-$C_{1-4}$-Alkyl,
(2) -OCOS-$C_{1-4}$-Alkyl, substituiert mit $\omega$-$C_{2-4}$-alkanoylamino,
(3) -OCOR$^{14}$, worin $R^{14}$ eine $C_{1-4}$-Alkylgruppe ist, die am Terminus durch (a) di($C_{1-4}$-Alkyl)amino oder (b) eine 5- bis 8-gliedrige heterozyklische Gruppe substituiert ist, die 1 bis 4 Heteroatome ausgewählt aus Sauerstoff, Schwefel oder Stickstoff enthält, und wahlweise ein dazwischenliegendes Schwefelatom aufweist,
(4) -OCONR$^{15}$R$^{16}$ ist, worin $R^{15}$ und $R^{16}$ jeweils eine $C_{1-5}$-Alkylgruppe sind oder gemeinsam eine heterozyklische Gruppe bilden, die Stickstoffatome enthält, die durch (1) $C_{1-4}$-Alkyl oder (2) Pyridyl substituiert sein können;

$R^4$ Hydroxy oder eine $C_{1-4}$-Alkanoyloxygruppe ist;
oder ein Salz davon, welches das Unterwerfen einer Verbindung der Formel:

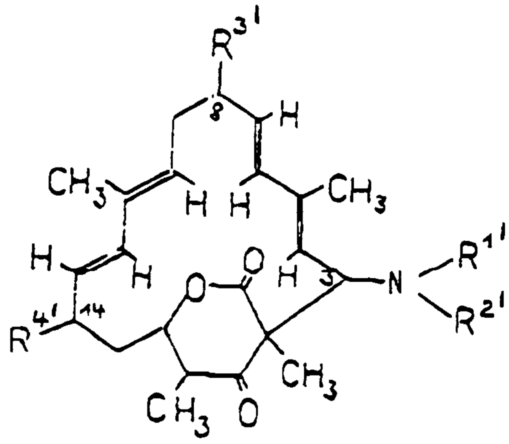

worin $R^{1'}$, $R^{2'}$, $R^{3'}$ und $R^{4'}$ jeweils die gleiche Bedeutung haben wie oben für $R^1$, $R^2$, $R^3$ und $R^4$ angegeben oder gleich den in sie umwandelbaren Gruppen sind, der Alkylierung, Acylierung, Hydrolyse, Thioamidierung, Halogenierung, Azidierung, Umsetzung mit Aminen, Umsetzung mit einem Thiol oder einem Salz und/oder Umsetzung mit Isocyanat umfaßt.

**2.** Verfahren nach Anspruch 1, worin Z Schwefel ist und $R^5$ Acetyl ist.

**3.** Verfahren nach Anspruch 1, worin Z Sauerstoff ist und $R^5$ Acetyl ist.

**4.** Verfahren nach Anspruch 1, worin das Salz ein Säureadditionssalz ist.

**5.** Verfahren nach Anspruch 1, worin $R^3$ -OCONR$^{15}$R$^{16}$ ist, worin $R^{15}$ und $R^{16}$ jeweils eine $C_{1-5}$-Alkylgruppe sind oder eine heterozyklische Gruppe bilden, die Stickstoffatome enthält, die durch (1) $C_{1-4}$-Alkyl oder (2) Pyridyl substituiert sein können.

**6.** Verfahren nach Anspruch 1, worin $R^4$ Hydroxy ist.

**7.** Verfahren nach Anspruch 1 zur Herstellung einer Verbindung, ausgewählt aus
3-(2-Oxo-1-thioxopropylamino)lankon 8-acetat,
O(8)-(4-Methylpiperazino)carbonyl-lankacidin A,
O(8)-[4-(2-Pyridyl)piperazino] carbonyl-lankacidin A,
Lankacidin C 8-dimethylaminoacetat,
Lankacidin C 8-diäthylaminoacetat,
Lankacidin A 8-[(2-dimethylaminoäthyl)thio]methylcarbonat,
Lankacidin C 8-[(2-dimethylaminoäthyl)thio]methylcarbonat,
O(8)-(4-methylpiperazino)carbonyl-lankacidin C,
Lankacidin A 8-[S-(2-dimethylaminoäthyl)thiocarbonat,
Lankacidin C 8-[S-(2-dimethylaminoäthyl)thiocarbonat,
Lankacidin A 8-diäthylaminoacetat,
Lankacidin A 8-diisopropylaminoacetat,
Lankacidin C 8-diiosypropylaminoacetat,
Lankacidin C 8-(3-dimethylamino)propionat,
Lankacidin C 8-(3-dimethylamino)butyrat,
Lankacidin C 8-morpholinoacetat,
O(8)-[4-(4-Pyridyl)piperazino)carbonyl-lankacidin C,
O(8)-[4-(2-Pyridyl)piperazino)carbonyl-lankacidin C,
oder eines Salzes davon.

**8.** Verfahren nach Anspruch 1 zur Herstellung einer Verbindung, ausgewählt aus
O(8)-(4-Benzylpiperazino)carbonyl-lankacidin C,
3-(2-Acetoxyacrylamido)lankon-8,14-diacetat
O(8)-(2-Acetamidoäthylthio)methoxycarbonyl-lankacidin C,

Lankacidin C 8-(2-chloräthyl)aminoacetat,
Lankacidin C 8-(2-fluoräthyl)aminoacetat,
O(8)-[2-(2-Pyridyl)äthylamino]carbonyl-lankacidin C,
O(8)-[(2-Pyridyl)methylamino]carbonyl-lankacidin C,
Lankacidin C 8-propylaminoacetat,
Lankacidin C 8-isopropylaminoacetat,
Lankacidin C 8-(4-(4-pyridyl)piperazino)acetat,
O(8)-[(5-Methoxymethyl-1,3,4,-thiadiazol-2-yl)thio]methoxycarbonyl-lankacidin C,
O(8)-(4-Pyridylmethylamino)carbonyl-lankacidin C,
Lankacidin C 8-azidoacetat,
8-Dehydroxy-8-(dimethylamino-1H-tetrazol-5-yl)thio-lankacidin C,
O(8)-[(1-Methyl-1H-tetrazol-5-yl)thio]methoxycarbonyl-lankacidin C,
Lankacidin C 8-[(1-methyl-1H-tetrazol-5-yl)thio]-acetat,
Lankacidin C 8-[4-(2-pyridyl)piperazino]acetat,
O(8)-Äthylaminocarbonyl-lankacidin C,
O(8)-Morpholinocarbonyl-lankacidin C,
O(8)-(3-Pyridyl)methylaminocarbonyl-lankacidin C,
oder eines Salzes davon.

## Revendications

**Revendications pour les Etats contractants suivants : BE, CH, DE, FR, GB, GR, IT, LU, LI, NL, SE**

**1.** Composé de formule générale :

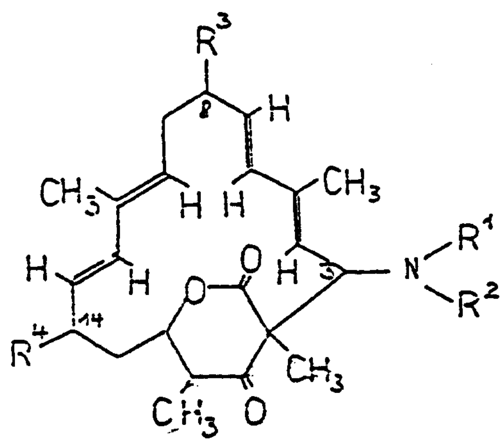

dans laquelle
(i) $R^1$ représente un atome d'hydrogène, $R^2$ représente un groupe de formule :

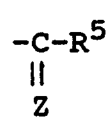

dans laquelle Z représente un atome d'oxygène ou de soufre et $R^5$ représente un groupe alcanoyle en $C_{1-5}$ ou un groupe $\alpha$-hydroxy-alkyle en $C_{1-4}$, ou bien
(ii) $R^1$ et $R^2$ représentent conjointement un groupe de formule :

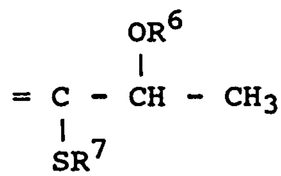

dans laquelle $R^6$ représente un groupe alcanoyle en $C_{1-5}$ et
$R^7$ représente un groupe alkyle en $C_{1-4}$ ;
$R^3$ représente
(1) un groupe -OCOO-alkyle en $C_{1-4}$,
(2) un groupe -OCOS-alkyle en $C_{1-4}$ substitué par un groupe $\omega$-alcanoylamino en $C_{2-4}$,
(3) un groupe -OCOR$^{14}$ dans lequel $R^{14}$ représente un groupe alkyle en $C_{1-4}$, substitué en son extrémité par (a) un groupe di(alkyle en $C_{1-4}$)-amino ou (b) un groupe hétérocyclique de 5 à 8 chaînons, comportant 1 à 4 hétéroatomes d'oxygène, de soufre ou d'azote, et comportant éventuellement un atome intermédiaire de soufre,
(4) un groupe -OCONR$^{15}$R$^{16}$ dans lequel $R^{15}$ et $R^{16}$ représentent chacun un groupe alkyle en $C_{1-5}$ ou forment conjointement un groupe hétérocyclique comportant des atomes d'azote, qui peut être substitué par (1) un groupe alkyle en $C_{1-4}$ ou (2) un groupe pyridyle ;
$R^4$ représente un groupe hydroxy ou un groupe alcanoyloxy en $C_{1-4}$ ;
ou un sel d'un tel composé.

**2.** Composé conforme à la revendication 1, dans lequel Z représente un atome de soufre et $R^5$ représente un groupe acétyle.

**3.** Composé conforme à la revendication 1, dans lequel Z représente un atome d'oxygène et $R^5$ représente un groupe acétyle.

**4.** Composé conforme à la revendication 1, pour lequel le sel est un sel d'addition d'acide.

**5.** Composé conforme à la revendication 1, dans lequel $R^3$ représente un groupe -OCONR$^{15}$R$^{16}$ dans lequel $R^{15}$ et $R^{16}$ représentent chacun un groupe alkyle en $C_{1-5}$ ou bien forment un groupe hétérocyclique comportant des atomes d'azote et qui peut être substitué par (1) un groupe alkyle en $C_{1-4}$ ou (2) un groupe pyridyle.

**6.** Composé conforme à la revendication 1, dans lequel $R^4$ représente un groupe hydroxy.

**7.** Un composé choisi parmi
le 8-acétate de 3-(2-oxo-1-thioxopropylamino)-lankone,
l'O(8)-(4-méthyl-pipérazino)carbonyl-lankacidine A,
l'O(8)-[4-(2-pyridyl)-pipérazino]carbonyl-lankacidine A,
le 8-diméthylamino-acétate de lankacidine C,
le 8-diméthylaminoacétate de lankacidine C,
le 8-[(2-diméthylaminoéthyl)thio)méthyl-carbonate de lankacidine A,
le 8-[(2-diméthylamino-éthyl)thio]méthylcarbonate de lankacidine C,
l'O(8)-(4-méthylpipérazino)carbonyl-lankacidine C,
le 8-[S-(2-diméthylaminoéthyl)thiocarbonate de lankacidine A,
le 8-[S-(2-diméthylaminoéthyl)thiocarbonate de lankacidine C,
le 8-diéthylaminoacétate de lankacidine A,
le 8-diisopropylaminoacétate de lankacidine A,
le 8-diisopropylaminoacétate de lankacidine C,
le 8-(3-diméthylamino)propionate de lankacidine C,
le 8-(3-diméthylamino)butyrate de lankacidine C,
le 8-morpholinoacétate de lankacidine C,
l'O(8)-[4-(4-pyridyl)pipérazino)carbonyl-lankacidine C,
l'O(8)-[4-(2-pyridyl)pipérazino)carbonyl-lankacidine C,
ou un sel d'un tel composé.

**8.** Un composé choisi parmi
l'O(8)-(4-benzylpipérazino)carbonyl-lankacidine C,
le 8,14-diacétate de 3-(2-acétoxyacrylamido)lankone,
l'O(8)-(2-acétamidoéthylthio)méthoxycarbonyl-lankacidine C,
le 8-(2-chloroéthyl)aminoacétate de lankacidine C,
le 8-(2-fluoroéthyl)aminoacétate de lankacidine C,
l'O(8)-[2-(2-pyridyl)éthylamino)carbonyl-lankacidine C,

l'O(8)-[2-(2-pyridyl)méthylamino]carbonyl-lankacidine C,
le 8-propylaminoacétate de lankacidine C,
le 8-isopropylaminoacétate de lankacidine C,
le 8-(4-(4-pyridyl)pipérazino)acétate de lankacidine C,
l'O(8)-[(5-méthoxyméthyl-1,3,4-thiadiazol-2-yl)thio]méthoxy-carbonyl-lankacidine C,
l'O(8)-(4-pyridylméthylamino)carbonyl-lankacidine C,
le 8-azidoacétate de lankacidine C,
la 8-déhydroxy-8-(diméthylamino-1H-tétrazol-5-yl)thiolankacidine C,
l'O(8)-[(1-méthyl-1H-tétrazol-5-yl)thio]méthoxycarbonyl-lankacidine C,
le 8-[(1-méthyl-1H-tétrazol-5-yl)thio]acétate de lankacidine C,
le 8-[4-(2-pyridyl)pipérazino]acétate de lankacidine C,
l'O(8)-éthylaminocarbonyl-lankacidine C,
l'O(8)-morpholinocarbonyl-lankacidine C,
l'O(8)-(3(pyridyl)méthylaminocarbonyl-lankacidine C,
ou un sel d'un tel composé.

**9.** Procédé de production d'un composé conforme à l'une des revendications 1 à 8, qui comporte le fait de soumettre un composé de formule :

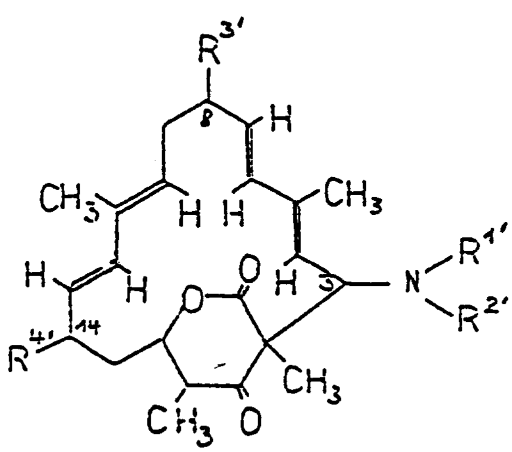

dans laquelle $R^{1'}$, $R^{2'}$, $R^{3'}$ et $R^{4'}$ sont respectivement identiques à $R^1$, $R^2$, $R^3$ et $R^4$ définis plus haut ou des groupes convertibles en ces derniers,
à une alkylation, une acylation, une hydrolyse, une thioamidation, une halogénation, une azidation, une réaction avec des amines, une réaction avec un thiol ou un sel, et/ou une réaction avec un isocyanate.

**Revendications pour les Etats contractants suivants : AT, ES**

**1.** Procédé de production d'un composé de formule générale :

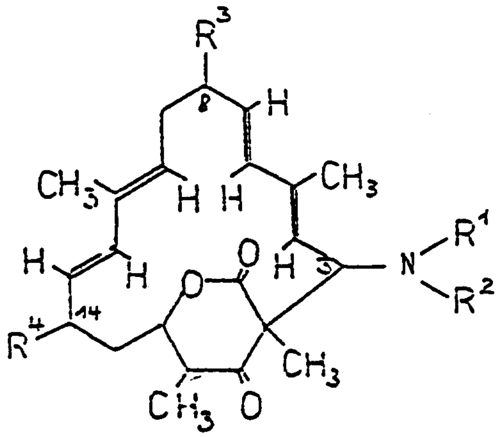

dans laquelle

(i) $R^1$ représente un atome d'hydrogène, $R^2$ représente un groupe de formule :

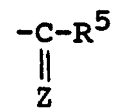

dans laquelle Z représente un atome d'oxygène ou de soufre et $R^5$ représente un groupe alcanoyle en $C_{1-5}$ ou un groupe $\alpha$-hydroxy-alkyle en $C_{1-4}$, ou bien

(ii) $R^1$ et $R^2$ représentent conjointement un groupe de formule :

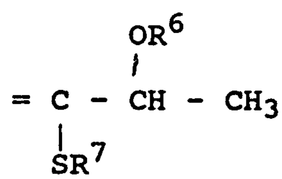

dans laquelle $R^6$ représente un groupe alcanoyle en $C_{1-5}$ et $R^7$ représente un groupe alkyle en $C_{1-4}$ ;

$R^3$ représente

(1) un groupe -OCOO-alkyle en $C_{1-4}$,

(2) un groupe -OCOS-alkyle en $C_{1-4}$ substitué par un groupe $\omega$-alcanoylamino en $C_{2-4}$,

(3) un groupe -$OCOR^{14}$ dans lequel $R^{14}$ représente un groupe alkyle en $C_{1-4}$, substitué en son extrémité par (a) un groupe di(alkyle en $C_{1-4}$)-amino ou (b) un groupe hétérocyclique de 5 à 8 chaînons, comportant 1 à 4 hétéroatomes d'oxygène, de soufre ou d'azote, et comportant éventuellement un atome intermédiaire de soufre,

(4) un groupe -$OCONR^{15}R^{16}$ dans lequel $R^{15}$ et $R^{16}$ représentent chacun un groupe alkyle en $C_{1-5}$ ou forment conjointement un groupe hétérocyclique comportant des atomes d'azote, qui peut être substitué par (1) un groupe alkyle en $C_{1-4}$ ou (2) un groupe pyridyle ;

$R^4$ représente un groupe hydroxy ou un groupe alcanoyloxy en $C_{1-4}$ ;

ou un sel d'un tel composé,

qui comporte le fait de soumettre un composé de formule :

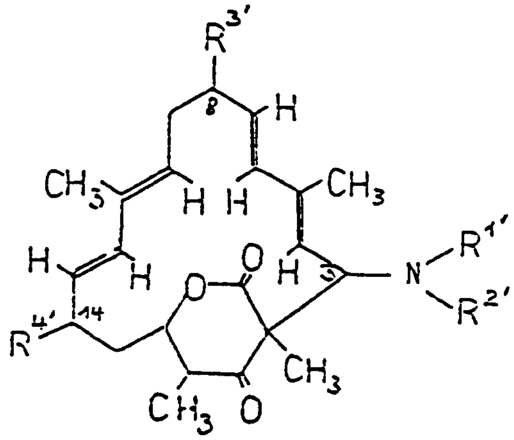

dans laquelle $R^{1'}$, $R^{2'}$, $R^{3'}$ et $R^{4'}$ sont respectivement identiques à $R^1$, $R^2$, $R^3$ et $R^4$ définis plus haut ou des groupes convertibles en ces derniers,
à une alkylation, une acylation, une hydrolyse, une thioamidation, une halogénation, une azidation, une réaction avec des amines, une réaction avec un thiol ou un sel, et/ou une réaction avec un isocyanate.

**2.** Procédé selon la revendication 1, dans lequel Z représente un atome de soufre et $R^5$ représente un groupe acétyle.

**3.** Procédé selon la revendication 1, dans lequel Z représente un atome d'oxygène et $R^5$ représente un groupe acétyle.

**4.** Procédé selon la revendication 1, dans lequel le sel est un sel d'addition d'acide.

**5.** Procédé selon la revendication 1, dans lequel $R^3$ représente un groupe $-OCONR^{15}R^{16}$ dans lequel $R^{15}$ et $R^{16}$ représentent chacun un groupe alkyle en $C_{1-5}$ ou forment conjointement un groupe hétérocyclique comportant des atomes d'azote, qui peut être substitué par (1) un groupe alkyle en $C_{1-4}$ ou (2) un groupe pyridyle.

**6.** Procédé selon la revendication 1, dans lequel $R^4$ est un groupe hydroxy.

**7.** Procédé selon la revendication 1, pour préparer un composé choisi parmi :
le 8-acétate de 3-(2-oxo-1-thioxopropylamino)-lankone,
l'O(8)-(4-méthyl-pipérazino)carbonyl-lankacidine A,
l'O(8)-[4-(2-pyridyl)-pipérazino]carbonyl-lankacidine A,
le 8-diméthylamino-acétate de lankacidine C,
le 8-diéthylaminoacétate de lankacidine C,
le 8-[(2-diméthylaminoéthyl)thio]méthyl-carbonate de lankacidine A,
le 8-[(2-diméthylamino-éthyl)thio]méthylcarbonate de lankacidine C,
l'O(8)-(4-méthylpipérazino)carbonyl-lankacidine C,
le 8-[S-(2-diméthylaminoéthyl)thiocarbonate de lankacidine A,
le 8-[S-(2-diméthylaminoéthyl)thiocarbonate de lankacidine C,
le 8-diéthylaminoacétate de lankacidine A,
le 8-diisopropylaminoacétate de lankacidine A,
le 8-diisopropylaminoacétate de lankacidine C,
le 8-(3-diméthylamino)propionate de lankacidine C,
le 8-(3-diméthylamino)butyrate de lankacidine C,
le 8-morpholinoacétate de lankacidine C,
l'O(8)-[4-(4-pyridyl)pipérazino)carbonyl-lankacidine C,
l'O(8)-[4-(2-pyridyl)pipérazino)carbonyl-lankacidine C,
ou un sel d'un tel composé.

**8.** Procédé selon la revendication 1, pour la préparation d'un composé choisi parmi
l'O(8)-(4-benzylpipérazino)carbonyl-lankacidine C,
le 8, 14-diacétate de 3-(2-acétoxyacrylamido)lankone,
l'O(8)-(2-acétamidoéthylthio)méthoxycarbonyl-lankacidine C,
le 8-(2-chloroéthyl)aminoacétate de lankacidine C,
le 8-(2-fluoroéthyl)aminoacétate de lankacidine C,
l'O(8)-[2-(2-pyridyl)éthylamino)carbonyl-lankacidine C,
l'O(8)-[2-(2-pyridyl)méthylamino)carbonyl-lankacidine C,
le 8-propylaminoacétate de lankacidine C,
le 8-isopropylaminoacétate de lankacidine C,
le 8-(4-(4-pyridyl)pipérazino)acétate de lankacidine C,
l'O(8)-[(5-méthoxyméthyl-1,3,4-thiadiazol-2-yl)thio]méthoxy-carbonyl-lankacidine C,
l'O(8)-(4-pyridylméthylamino)carbonyl-lankacidine C,
le 8-azidoacétate de lankacidine C,
la 8-déhydroxy-8-(diméthylamino-1H-tétrazol-5-yl)thio-lankacidine C,
l'O(8)-[(1-méthyl-1H-tétrazol-5-yl)thio]méthoxycarbonyl-lankacidine C,
le 8-[(1-méthyl-1H-tétrazol-5-yl)thio]acétate de lankacidine C,
le 8-[4-(2-pyridyl)pipérazino]acétate de lankacidine C,
l'O(8)-éthylaminocarbonyl-lankacidine C,
l'O(8)-morpholinocarbonyl-lankacidine C,
l'O(8)-(3(pyridyl)méthylaminocarbonyl-lankacidine C,
ou d'un sel d'un tel composé.